(19) Europäisches Patentamt
European Patent Office
Office européen des brevets

(11) EP 3 584 318 A1

(12) **EUROPEAN PATENT APPLICATION**
published in accordance with Art. 153(4) EPC

(43) Date of publication:
**25.12.2019 Bulletin 2019/52**

(21) Application number: **18753804.6**

(22) Date of filing: **15.02.2018**

(51) Int Cl.:
***C12N 11/10*** *(2006.01)* ***C12N 1/00*** *(2006.01)*

(86) International application number:
**PCT/JP2018/005156**

(87) International publication number:
**WO 2018/151186 (23.08.2018 Gazette 2018/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **16.02.2017 JP 2017027246**
**24.11.2017 JP 2017225903**

(71) Applicant: **Ajinomoto Co., Inc.**
**Tokyo 104-8315 (JP)**

(72) Inventors:
- **OHSUMI, Koji**
**Kawasaki-shi**
**Kanagawa 210-8681 (JP)**
- **FUJII, Tomohiro**
**Kawasaki-shi**
**Kanagawa 210-8681 (JP)**

(74) Representative: **Hoffmann Eitle**
**Patent- und Rechtsanwälte PartmbB**
**Arabellastraße 30**
**81925 München (DE)**

(54) **POLYMER BEAD**

(57) The present invention provides a bead containing a polymer containing uronic acid units having mercapto groups, the mercapto groups partly or entirely forming a disulfide bond.

EP 3 584 318 A1

## Description

[Technical Field]

The present invention relates to a polymer bead useful for enclosing a cell or a microorganism in the inside.

[Background Art]

In recent years, transplantation of a cell or the like encapsulated in a polymer bead or polymer capsule to the body has been studied. As such capsule, for example, non-patent document 1 describes an alginate/poly-L-orthinine/alginate capsule having a structure in which a core composed of alginate is coated with poly-L-orthinine and further coated with alginate.

Non-patent document 2 describes disulfide-crosslinked polygalacturonic acid hydrogel. Non-patent document 2 describes a film formed from the hydrogel but does not describe a bead or capsule formed from the hydrogel.

Non-patent document 3 describes a hydrogel of alginate bonded to an amine having an inhibitory action on a foreign-body reaction.

[Document List]

[non-patent document]

non-patent document 1: JOURNAL OF BIOMEDICAL MATERIALS RESEARCH B: APPLIED BIOMATERIALS I FEB 2013 VOL 101B, ISSUE 2, 258-268
non-patent document 2: J Mater Sci: Mater Med (2013) 24: 1375-1382
non-patent document 3: Nature Biotechnology, Vol 34, No. 3 (2016), 345-352

[SUMMARY OF THE INVENTION]

[Problems to be Solved by the Invention]

The alginate (the second layer)/poly-L-orthinine (the first layer)/alginate capsule described in non-patent document 1 has a problem that the alginate layer in the second layer is decomposed in vivo to expose poly-L-orthinine in the first layer, as a result of which an inflammation reaction occurs. The present invention has been made by taking note of such circumstances and an object thereof is to provide a polymer bead superior in durability.

[Means of Solving the Problems]

The present inventors have conducted intensive studies in an attempt to achieve the above-mentioned object and found that a bead formed from a polymer containing uronic acid units having mercapto groups (-SH) has a disulfide bond (-S-S-) formed partly or entirely by the mercapto groups therein and shows superior durability even under low calcium ion conditions. The present invention based on this finding is as follows.

[1] A bead comprising a polymer comprising uronic acid units having mercapto groups, the mercapto groups partly or entirely forming a disulfide bond.
[2] The bead of the aforementioned [1] wherein the uronic acid unit having the mercapto group comprises a uronic acid residue and a residue of a compound represented by the formula (A1):

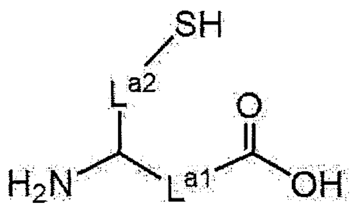

(A1)

wherein $L^{a1}$ is a single bond or a $C_{1-3}$ alkylene group and $La^2$ is a $C_{1-4}$ alkylene group, or a compound represented

by the formula (A2) :

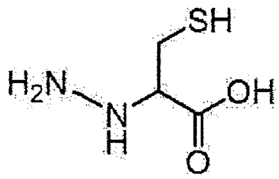 (A2)

bonded to each other via an amide bond.

[3] The bead of the aforementioned [1] or [2] wherein the uronic acid is at least one selected from the group consisting of galacturonic acid, mannuronic acid and guluronic acid.

[4] The bead of the aforementioned [1] or [2] wherein the uronic acid is at least one selected from the group consisting of mannuronic acid and guluronic acid, and the polymer comprising the uronic acid units having the mercapto groups is alginic acid having mercapto groups.

[5] The bead of the aforementioned [1] wherein the polymer comprising the uronic acid units having the mercapto groups is a polymer comprising galacturonic acid units having mercapto groups.

[6] The bead of the aforementioned [5] wherein the galacturonic acid unit having the mercapto group comprises a galacturonic acid residue and an amino acid residue having a mercapto group bonded to each other via an amide bond.

[7] The bead of the aforementioned [6] wherein the amino acid having the mercapto group is cysteine.

[8] The bead of any one of the aforementioned [5] to [7] wherein the polymer comprising the galacturonic acid units is polygalacturonic acid.

[9] The bead of any one of the aforementioned [1] to [8] wherein the polymer comprising the uronic acid units having the mercapto groups is further bonded to a compound represented by the formula (B):

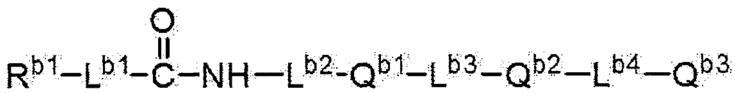 (B)

wherein $R^{b1}$ is a functional group capable of bonding to a mercapto group,

$L^{b1}$ and $L^{b2}$ are each independently a single bond or a $C_{1-6}$ alkylene group,

$Q^{b1}$ is a single bond, a phenylene group, or a $C_{4-8}$ cycloalkanediyl group,

$L^{b3}$ is a single bond or $*-(OCH_2CH_2)_n-**$ (wherein * shows a bonding position to $Q^{b1}$, ** shows a bonding position to $Q^{b2}$, and n is an integer of 1 - 10),

$Q^{b2}$ is a divalent triazole ring group,

$L^{b4}$ is a single bond, a $C_{1-6}$ alkylene group, or a $C_{1-6}$ alkylene-oxy group, and

$Q^{b3}$ is an optionally substituted phenyl group, an optionally substituted monovalent 5- or 6-membered heterocyclic group, or an optionally substituted $C_{4-8}$ cycloalkyl group.

[10] The bead of the aforementioned [9] wherein the compound represented by the formula (B) is at least one selected from the group consisting of a compound represented by the formula (B1) :

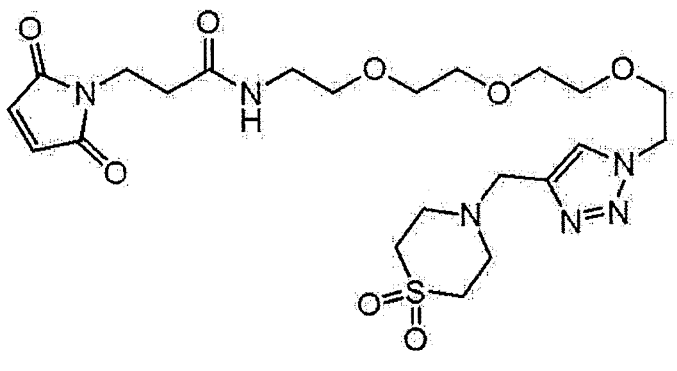 (B1)

,

a compound represented by the formula (B2):

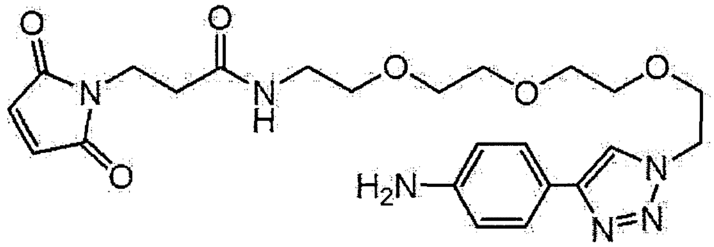

(B2)

,

and
a compound represented by the formula (B3):

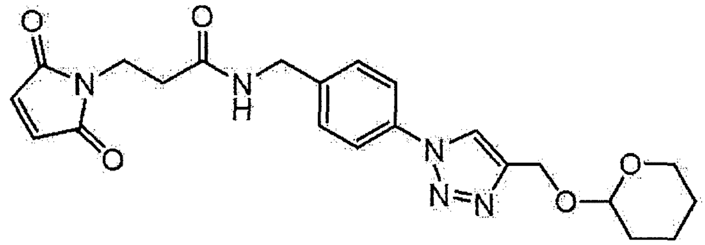

(B3)

.

[11] The bead of the aforementioned [9] wherein the compound represented by the formula (B) is a compound represented by the formula (B1):

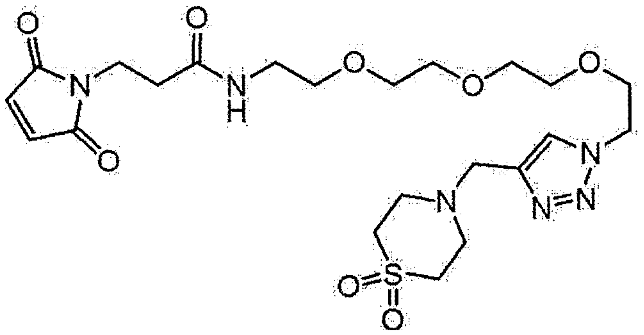

(B1)

.

[12] The bead of any one of the aforementioned [9] to [11] wherein the compound represented by the formula (B) is a compound having an inhibitory action on a foreign-body reaction.
[13] The bead of any one of the aforementioned [1] to [12] wherein a proportion of the uronic acid unit having the mercapto group in the total constitutional units of the polymer comprising the uronic acid units having the mercapto groups is 0.1 - 50 mol%.
[14] The bead of any one of the aforementioned [1] to [13] wherein a proportion of the mercapto group forming the disulfide bond in the total mercapto groups is 10 - 100 mol%.
[15] The bead of any one of the aforementioned [1] to [14] wherein a number average molecular weight of the polymer comprising the uronic acid units having the mercapto groups is 25,000 - 500,000.
[16] The bead of any one of the aforementioned [1] to [15] wherein the polymer comprises a divalent metal ion.
[17] The bead of the aforementioned [16] wherein the divalent metal ion is at least one selected from the group consisting of calcium ion, barium ion and strontium ion.
[18] The bead of any one of the aforementioned [1] to [17] further comprising a first layer and a second layer as outer layers, wherein the first layer is formed on the bead and the second layer is formed on the first layer.
[19] The bead of the aforementioned [18] wherein the uronic acid is at least one selected from the group consisting of mannuronic acid and guluronic acid,

the polymer comprising the uronic acid units having the mercapto groups is alginic acid having mercapto groups, and
the uronic acid unit having the mercapto group comprises a uronic acid residue and a residue of a compound represented by the formula (A1):

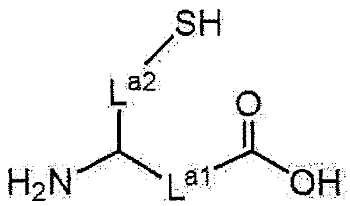

(A1)

wherein $L^{a1}$ is a single bond or a $C_{1-3}$ alkylene group and $L^{a2}$ is a $C_{1-4}$ alkylene group, or a compound represented by the formula (A2) :

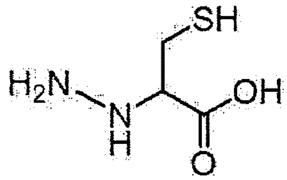

(A2)

bonded to each other via an amide bond.

[20] The bead of the aforementioned [19] wherein the compound represented by the formula (A1) or the compound represented by the formula (A2) is cysteine.

[21] The bead of any one of the aforementioned [18] to [20] wherein the first layer is formed from at least one selected from the group consisting of water-soluble chitosan and polyornithine.

[22] The bead of any one of the aforementioned [18] to [21] wherein the second layer is formed from at least one selected from the group consisting of polygalacturonic acid and polygalacturonic acid having mercapto groups.

[23] The bead of the aforementioned [22] wherein said at least one selected from the group consisting of the polygalacturonic acid and polygalacturonic acid having mercapto groups is further bonded to a compound represented by the formula (b):

$$H_2N\text{-}L^{b2}\text{-}Q^{b1}\text{-}L^{b3}\text{-}Q^{b2}\text{-}L^{b4}\text{-}Q^{b3} \qquad \text{(b)}$$

wherein $L^{b2}$ is a single bond or a $C_{1-6}$ alkylene group,
$Q^{b1}$ is a single bond, a phenylene group, or a $C_{4-8}$ cycloalkanediyl group,
$L^{b3}$ is a single bond or $*\text{-}(OCH_2CH_2)_n\text{-}**$ (wherein * shows a bonding position to $Q^{b1}$, ** shows a bonding position to $Q^{b2}$, and n is an integer of 1 - 10),
$Q^{b2}$ is a divalent triazole ring group,
$L^{b4}$ is a single bond, a $C_{1-6}$ alkylene group, or a $C_{1-6}$ alkylene-oxy group, and
$Q^{b3}$ is an optionally substituted phenyl group, an optionally substituted monovalent 5- or 6-membered heterocyclic group, or an optionally substituted $C_{4-8}$ cycloalkyl group.

[24] The bead of the aforementioned [23] wherein the compound represented by the formula (b) is at least one selected from the group consisting of a compound represented by the formula (b1) :

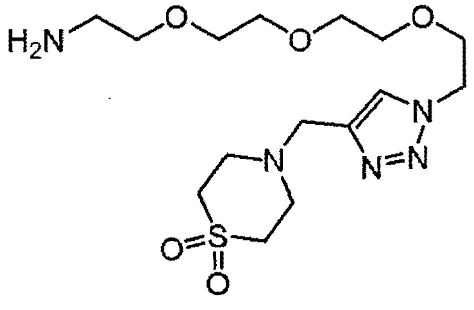

(b1)

,

a compound represented by the formula (b2):

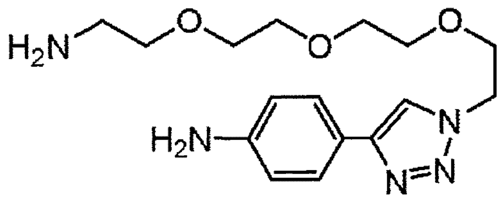

(b2)

,

and

a compound represented by the formula (b3):

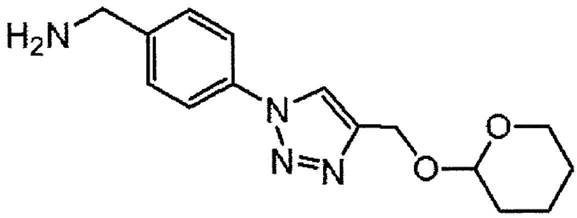

(b3)

.

[25] The bead of the aforementioned [23] wherein the compound represented by the formula (b) is a compound represented by the formula (b1):

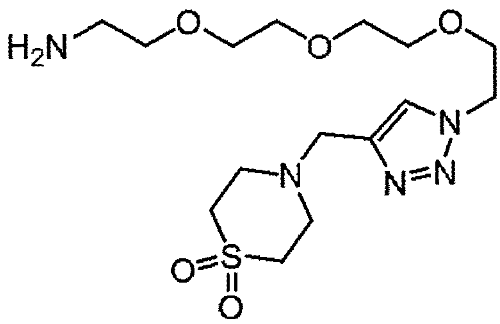

(b1)

.

[26] The bead of any one of the aforementioned [23] to [25] wherein the compound represented by the formula (b) is a compound having an inhibitory action on a foreign-body reaction.

[27] The bead of any one of the aforementioned [1] to [26] for use for enclosing a cell or a microorganism in the inside.

[28] The bead of any one of the aforementioned [1] to [26] enclosing the cell or microorganism in the inside.

[Effect of the Invention]

According to the present invention, a polymer bead superior in durability can be obtained.

[Description of Embodiments]

The present invention provides a bead having a polymer containing uronic acid units having mercapto groups (hereinafter sometimes to be referred to as "mercapto group-containing uronic acid-based polymer") and having a disulfide bond formed partly or entirely by the mercapto groups. The bead of the present invention is preferably used for enclosing a cell or microorganism in the inside thereof. Thus, the present invention also provides a bead enclosing a cell or microorganism in the inside.

The "uronic acid" means a carboxylic acid obtained by conversion of a hydroxymethyl group ($-CH_2OH$) of monosaccharide to a carboxy group by oxidation. Examples thereof include galacturonic acid, mannuronic acid, guluronic acid, arabinonic acid, fructuronic acid, tagaturonic acid, glucuronic acid, iduronic acid and the like. Only one kind or two or more kinds of the uronic acid may be used. As the uronic acid, galacturonic acid, mannuronic acid or guluronic acid is preferable, and galacturonic acid is more preferable.

In the present invention, the "bead" means an internally-filled sphere. In the pertinent field, a bead used for enclosing a cell or microorganism in the inside is sometimes called a "capsule".

Examples of the cell to be encapsulated in the bead of the present invention include cells for transplantation

and cells for culture. Examples of the cell for transplantation include cells derived from mammals. Examples of the cell derived from mammal include cells derived from human and cells derived from swine. Examples of the cell derived from human and cell derived from swine include hormone secreting cells thereof. Examples of the hormone secreting cell include pancreatic cell and pituitary cell. Examples of the cell for culture include stem cells such as iPS cell (induced pluripotent stem cell), ES cell (embryonic stem cell), MSC cell (mesenchymal stem cell) and the like. The microorganism to be encapsulated in the bead of the present invention may be any of aerobic bacteria and anaerobic bacteria.

The bead of the present invention may contain a polymer other than a mercapto group-containing uronic acid-based polymer. Examples of such other polymer include alginic acid free of a mercapto group and a salt thereof (i.e., alginate), chitosan, hyaluronic acid, gelatin, carboxymethylcellulose, gellan gum, glucomannan and the like. Only one kind or two or more kinds of such other polymers may be used. As the other polymer, alginic acid free of a mercapto group or a salt thereof is preferable, and alginate free of a mercapto group is more preferable. When the bead of the present invention contains other polymer, the amount thereof is preferably 1 - 80 wt%, more preferably 10 - 70 wt%, further preferably 20 - 60 wt%, per total polymers contained in the bead. The bead of the present invention particularly preferably contains a mercapto group-containing uronic acid-based polymer alone as a polymer constituting the bead. That is, polymer constituting the bead is particularly preferably composed of a mercapto group-containing uronic acid-based polymer.

Only one kind or two or more kinds of the polymer containing uronic acid units (i.e., polymer constituting the main chain of the mercapto group-containing uronic acid-based polymer) may be used. The polymer containing uronic acid units is preferably at least one selected from the group consisting of a galacturonic acid unit, a mannuronic acid unit and a guluronic acid unit, more preferably at least one selected from the group consisting of a polymer containing galacturonic acid units and a polymer containing mannuronic acid units and guluronic acid units, further preferably a polymer containing galacturonic acid units.

Examples of the polymer containing the galacturonic acid units include polygalacturonic acid, pectin (i.e., polymer in which carboxy groups of polygalacturonic acid are partly methylesterified) and the like. A preferable polymer containing uronic acid units having mercapto groups is, for example, a polymer containing galacturonic acid units having mercapto groups. Only one kind or two or more kinds of the aforementioned polymer may be used. The aforementioned polymer is more preferably polygalacturonic acid having mercapto groups.

A preferable polymer containing mannuronic acid units and guluronic acid units is, for example, alginic acid. That is, a preferable polymer containing uronic acid units having mercapto groups is, for example, alginic acid having mercapto groups.

The uronic acid unit having a mercapto group is preferably one in which a uronic acid residue and a residue of a compound represented by the formula (A1):

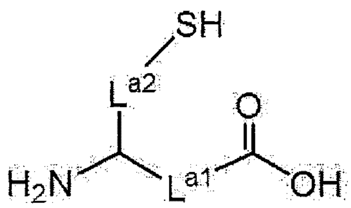

(A1)

wherein $L^{a1}$ is a single bond or a $C_{1-3}$ alkylene group, and $L^{a2}$ is a $C_{1-4}$ alkylene group, or a compound represented by the formula (A2) :

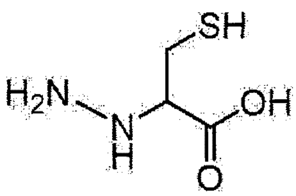

(A2)

are bonded via an amide bond (peptide bond). In the following, "compound represented by the formula (A1)" is sometimes to be abbreviated as "compound (A1)". Also, compounds represented by other formulas are sometimes to be abbreviated similarly. In addition, compound (A1) and compound (A2) are sometimes collectively indicated as "compound (A)".

Only one kind or two or more kinds of the uronic acid units having mercapto groups may be used. Therefore, the mercapto group-containing uronic acid-based polymer may have, as the uronic acid units having mercapto groups, only a unit constituted of a uronic acid residue and a residue of compound (A1), or only a unit constituted of a uronic

acid residue and a residue of compound (A2), or both a unit constituted of a uronic acid residue and a residue of compound (A1) and a unit constituted of a uronic acid residue and a residue of compound (A2). Only one kind or two or more kinds of compound (A1) may be used.

The carboxy group of the mercapto group-containing uronic acid-based polymer contributes to the water-solubility of the polymer and maintenance of the strength of the obtained bead. Therefore, it is preferable to use the above-mentioned compound (A) having both the mercapto group and the carboxy group to introduce a mercapto group into a polymer containing uronic acid units without reducing the amount of the carboxy group.

The aforementioned amide bond is preferably formed from a carboxy group in a uronic acid unit and an amino group of compound (A). That is, the mercapto group-containing uronic acid-based polymer is preferably a polymer formed by direct bonding of the amino group of compound (A) and the carboxy group of the polymer containing uronic acid units. A mercapto group-containing uronic acid-based polymer having directly bonded compound (A) shows less steric hindrance compared to a mercapto group-containing uronic acid-based polymer in which compound (A) is bonded via a linker such as polyethylene glycol chain or the like and does not inhibit gelling of polymers, thus producing a stronger bead.

In the present specification, the "$C_{x-y}$" means that the carbon atom number is not less than x and not more than y (x, y: integer).

In the present specification, the alkylene group may be linear or branched chain. The alkylene group is preferably linear. In the present specification, examples of the "$C_{1-6}$ alkylene group" include $-CH_2-$, $-(CH_2)_2-$, $-(CH_2)_3-$, $-(CH_2)_4-$, $-(CH_2)_5-$, $-(CH_2)_6-$, $-CH(CH_3)-$, $-C(CH_3)_2-$, $-CH(C_2H_5)-$, $-CH(C_3H_7)-$, $-CH(CH(CH_3)_2)-$, $-(CH(CH_3))_2-$, $-CH_2-CH(CH_3)-$, $-CH(CH_3)-CH_2-$, $-CH_2-CH_2-C(CH_3)_2-$, $-C(CH_3)_2-CH_2-CH_2-$, $-CH_2-CH_2-CH_2-C(CH_3)_2-$, and $-C(CH_3)_2-CH_2-CH_2-CH_2-$. In the present specification, examples of the "$C_{1-2}$ alkylene group", "$C_{1-3}$ alkylene group" and "$C_{1-4}$ alkylene group" respectively include those mentioned above and having 1 - 2 carbon atoms, 1 - 3 carbon atoms and 1 - 4 carbon atoms.

$L^{a1}$ is preferably a single bond or a $C_{1-2}$alkylene group, more preferably a single bond or $-CH_2-$, further preferably a single bond.

$La^2$ is preferably a $C_{1-3}$ alkylene group, more preferably a $C_{1-2}$ alkylene group, further preferably $-CH_2-$.

Specific examples of compound (A1) (i.e., amino acid having mercapto group) include the following.

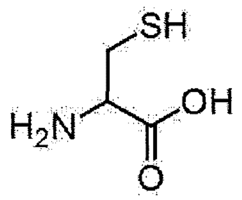
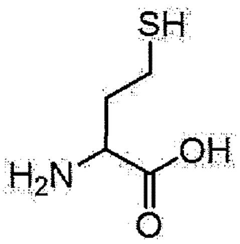
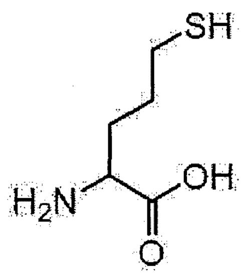
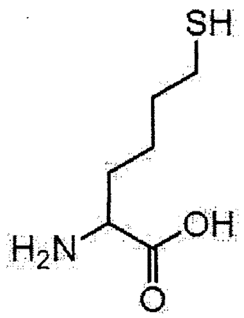

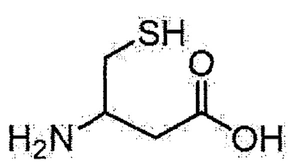
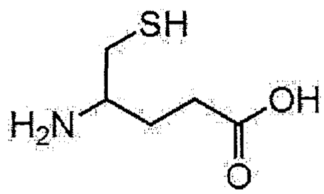
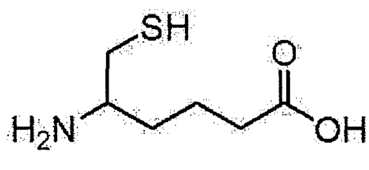

Compound (A1) is available from, for example, KANTO CHEMICAL CO., INC., FCH Group and the like. Compound (A2) is available from FCH Group and the like. Of compound (A1) and compound (A2), compound (A1) is preferable, cysteine and homocysteine are more preferable, and cysteine is further preferable.

A carboxy group of the polymer containing uronic acid units can be condensed with an amino group of compound (A) under conditions well known to those of ordinary skill in the art. The aforementioned polymer can be produced, for example, according to the method described non-patent document 2.

It is preferable to use a condensing agent for the aforementioned condensation of the carboxy group and the amino group. Examples of the condensing agent include 1-ethyl-3-(3-dimethylaminopropyl)carbodiimide hydrochloride (EDC-HCl) and the like. When EDC-HCl is used, N-hydroxysulfosuccinimide sodium is preferably used in combination.

After the aforementioned condensation, the reaction mixture is purified by a known means such as dialysis and the like, whereby a mercapto group-containing uronic acid-based polymer is preferably obtained.

A polymer containing uronic acid units having mercapto groups is preferably a polymer containing galacturonic acid units having mercapto groups, more preferably polygalacturonic acid having mercapto groups. In these embodiments, the galacturonic acid unit having a mercapto group more preferably has a galacturonic acid residue and an amino acid residue having a mercapto group bonded to each other via an amide bond (peptide bond).

The aforementioned amide bond is more preferably formed from a carboxy group in the galacturonic acid unit and an amino group of an amino acid having a mercapto group. That is, a polymer containing galacturonic acid units having mercapto groups is further preferably a polymer formed from an amino group of an amino acid having a mercapto group and a carboxy group of a polymer containing galacturonic acid units directly bonded to each other.

A carboxy group of a polymer containing galacturonic acid units can be condensed with an amino group of amino acid under conditions well known to those of ordinary skill in the art. The aforementioned polymer can be produced, for example, according to the method described non-patent document 2.

Specific examples of the amino acid having a mercapto group include the aforementioned specific examples of compound (A1). Only one kind or two or more kinds of amino acid having a mercapto group may be used. The amino acid having a mercapto group is preferably at least one selected from the group consisting of cysteine and homocysteine, more preferably cysteine.

The mercapto group-containing uronic acid-based polymer is further preferably bonded to a compound represented by the formula (B):

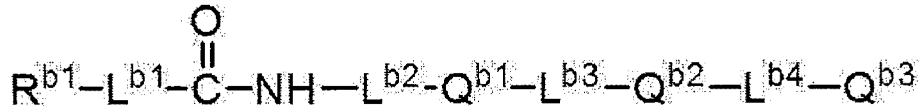 (B)

wherein $R^{b1}$ is a functional group capable of bonding to a mercapto group,
$L^{b1}$ and $L^{b2}$ are each independently a single bond or a $C_{1-6}$ alkylene group,
$Q^{b1}$ is a single bond, a phenylene group, or a $C_{4-8}$ cycloalkanediyl group,
$L^{b3}$ is a single bond or $*\text{-}(OCH_2CH_2)_n\text{-}**$ (wherein * shows a bonding position to $Q^{b1}$, ** shows a bonding position to $Q^{b2}$, and n is an integer of 1 - 100),
$Q^{b2}$ is a divalent triazole ring group,
$L^{b4}$ is a single bond, a $C_{1-6}$ alkylene group, or a $C_{1-6}$ alkylene-oxy group, and
$Q^{b3}$ is an optionally substituted phenyl group, an optionally substituted monovalent 5- or 6-membered heterocyclic group, or an optionally substituted $C_{4-8}$ cycloalkyl group.

In the present specification, examples of the "functional group capable of bonding to a mercapto group" include 1-maleimidyl group, chloromethylcarbonyl group, bromomethylcarbonyl group, vinylsulfonyl group, (meth)acryloyl group, disulfide bond, and organic groups containing any of these groups. Specific examples of the functional group capable of bonding to a mercapto group include the following (in the following formulas, * shows a bonding position).

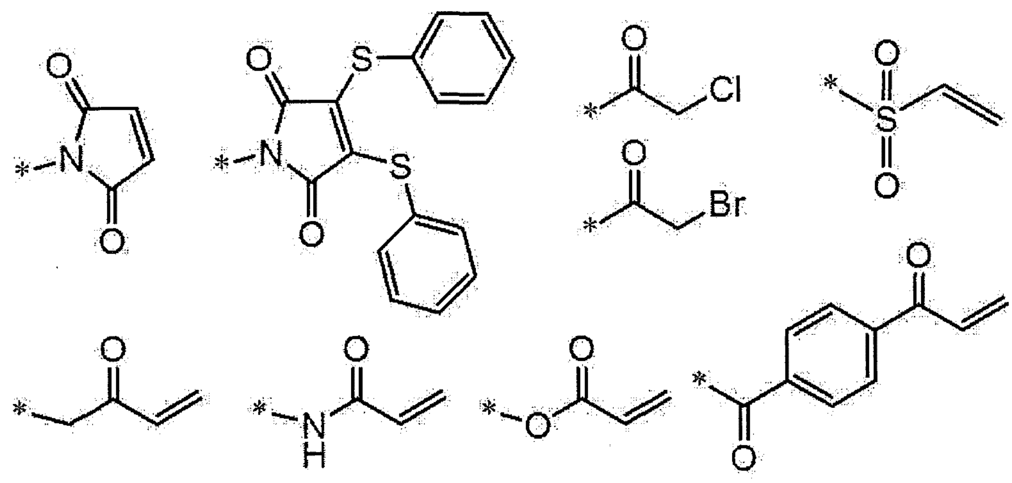

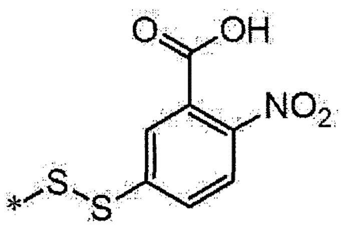 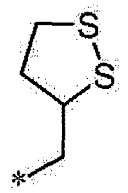

In the present specification, examples of the "$C_{4-6}$ cycloalkyl group" include cyclobutyl group, cyclopentyl group, cyclohexyl group, cycloheptyl group, and cyclooctyl group.

In the present specification, examples of the "$C_{4-8}$ cycloalkanediyl group" include cyclobutane-1,3-diyl group, cyclopentane-1,3-diyl group, cyclohexane-1,4-diyl group, cycloheptane-1,4-diyl group, and cyclooctane-1,5-diyl group.

In the present specification, examples of the "monovalent 5- or 6-membered heterocyclic group" include monovalent groups formed by removing one hydrogen bond from the following heterocycles.

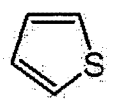 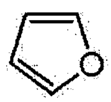 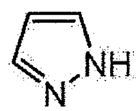 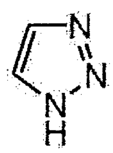 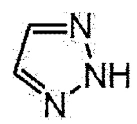

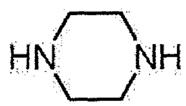 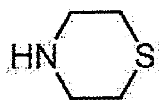 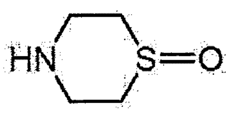 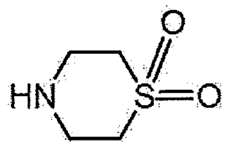

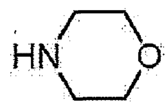 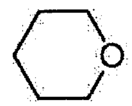 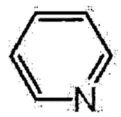

In the present specification, the "divalent triazole ring group" means a divalent monovalent groups formed by removing two hydrogen bonds from a triazole ring.

In the present specification, examples of the substituent that the phenyl group optionally has include halogen atom and amino group.

In the present specification, examples of the substituent that the monovalent 5- or 6-membered heterocyclic group optionally has include halogen atom and oxo group.

In the present specification, examples of the substituent that the $C_{4-8}$ cycloalkyl group optionally has include halogen atom and oxo group.

$R^{b1}$ is preferably a 1-maleimidyl group.

$L^{b1}$ is preferably $-(CH_2)_2-$.

$L^{b2}$ is preferably $-CH_2-$ or $-(CH_2)_2-$, more preferably - $(CH_2)_2-$.

$Q^{b1}$ is preferably a single bond or a 1,4-phenylene group, more preferably a single bond.

The n in $*-(OCH_2CH_2)_n-**$ for $L^{b3}$ is preferably an integer of 1 - 6, more preferably 3. $L^{b3}$ is preferably a single bond or $*-(OCH_2CH_2)_n-**$ (wherein n is an integer of 1 - 6), more preferably a single bond or $*-(OCH_2CH_2)_3-**$, further preferably $*-(OCH_2CH_2)_3-**$.

$Q^{b2}$ is preferably a 1H-1,2,3-triazole-1,4-diyl group.

$L^{b4}$ is preferably a single bond, $-CH_2-$ or $-CH_2-O-$, more preferably a single bond or $-CH_2-$, further preferably $-CH_2-$.

$Q^{b3}$ is preferably a 1,1-dioxothiomorpholin-4-yl group, a 4-aminophenyl group or a tetrahydropyran-2-yl group, more preferably a 1,1-dioxothiomorpholin-4-yl group or a 4-aminophenyl group, further preferably a 1,1-dioxothiomorpholin-4-yl group.

Compound (B) is preferably at least one selected from the group consisting of compound (B1), compound (B2) and compound (B3), which are represented by the following formulas, more preferably compound (B1).

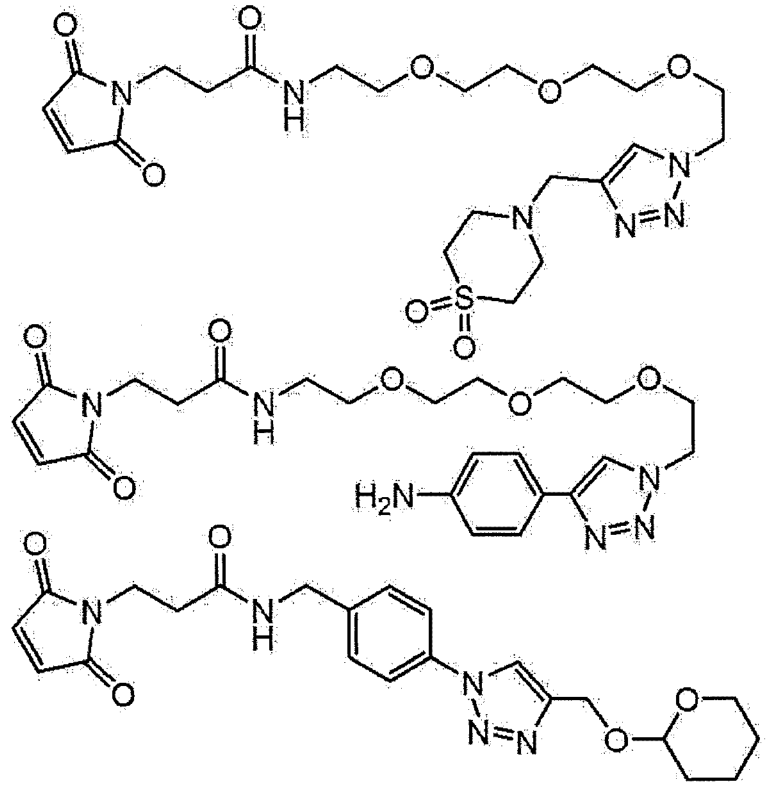

(B1)

(B2)

(B3)

Compound (B1) is a compound of the formula (B) wherein $R^{b1}$ is a 1-maleimidyl group, $L^{b1}$ is $-(CH_2)_2-$, $L^{b2}$ is $-(CH_2)_2$ $Q^{b1}$ is a single bond, $L^{b3}$ is $*-(OCH_2CH_2)_3-**$, $Q^{b2}$ is a 1H-1,2,3-triazole-1,4-diyl group, $L^{b4}$ is $-CH_2-$ and $Q^{b3}$ is a 1,1-dioxothiomorpholin-4-yl group.

Compound (B2) is a compound of the formula (B) wherein $R^{b1}$ is a 1-maleimidyl group, $L^{b1}$ is $-(CH_2)_2-$, $L^{b2}$ is $-(CH_2)_2-$, $Q^{b1}$ is a single bond, $L^{b3}$ is $*-(OCH_2CH_2)_3-**$, $Q^{b2}$ is a 1H-1,2,3-triazole-1,4-diyl group, $L^{b4}$ is a single bond and $Q^{b3}$ is a 4-aminophenyl group.

Compound (B3) is a compound of the formula (B) wherein $R^{b1}$ is a 1-maleimidyl group, $L^{b1}$ is - $(CH_2)_2-$, $L^{b2}$ is $-CH_2-$, $Q^{b1}$ is a 1,4-phenylene group, $L^{b3}$ is a single bond, $Q^{b2}$ is a 1H-1,2,3-triazole-1,4-diyl group, $L^{b4}$ is $-CH_2-O-$, and $Q^{b3}$ is a tetrahydropyran-2-yl group.

Compound (B) is preferably a compound having an inhibitory action on a foreign-body reaction. As used herein, the "inhibitory action on foreign-body reaction" means an action to inhibit adhesion of an inflammatory cell to a surface of a bead and an action to inhibit formation of a fibrous layer after death of the inflammatory cell adhered to a surface of a bead. Examples of the compound having an inhibitory action on a foreign-body reaction include the aforementioned compound (B1) to compound (B3).

Compound (B1) can be produced as described in the below-mentioned Production Examples 7 and 8. Compound (B2) and compound (B3) can be produced according to non-patent document 3 (particularly experiment therein) and in the same manner as in compound (B1) except that amine having the corresponding inhibitory action on a foreign-body reaction is prepared. Compound (B) can be produced by, for example, the following reactions (in the following formula, X is a leaving group (e.g., halogen atom, substituted or unsubstituted phenyloxy group, maleimidyloxy group etc.), and other symbols are as defined above).

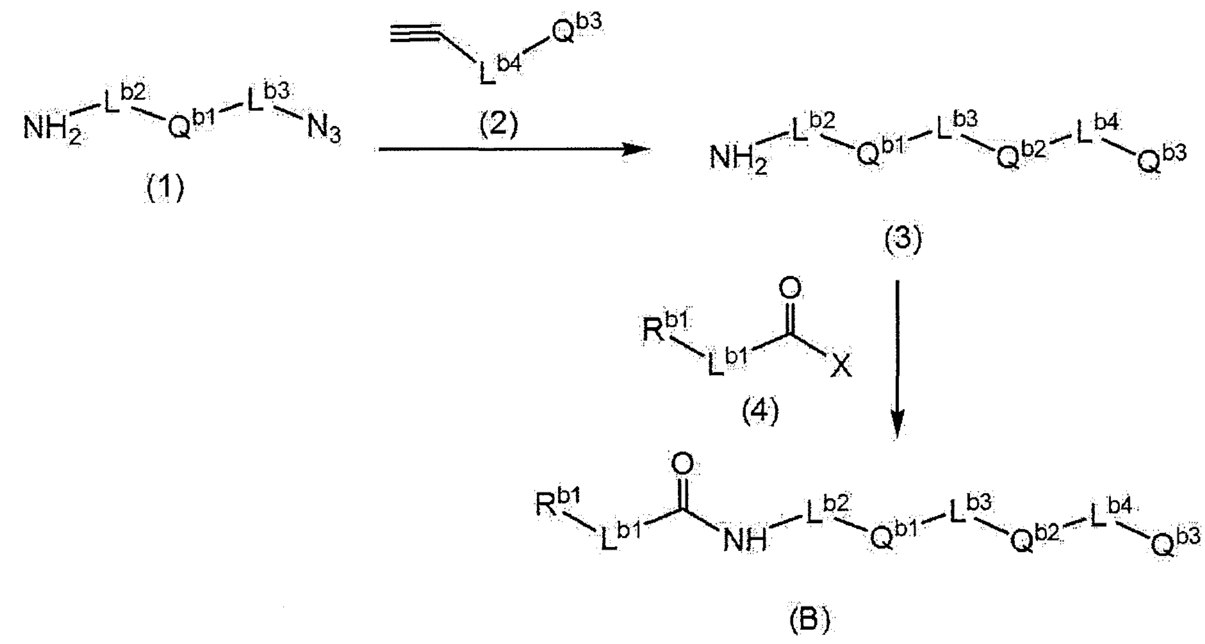

(1) (2) (3) (4) (B)

Compound (3) can be conveniently synthesized by a Click reaction of compound (1) having an azido group ($-N_3$) and compound (2) having an ethynyl group (-C≡CH) in water or an organic solvent. Compound (3) can be purified by silica gel chromatography and the like. Then, compound (3) is reacted with compound (4) having a leaving group X to synthesize compound (B). Compound (B) can be purified by silica gel chromatography and the like.

Compound (B) wherein $R^{b1}$ is a 1-maleimidyl group or organic group containing a 1-maleimidyl group shows high reactivity with a mercapto group. Thus, a bead can be bonded to the aforementioned compound (B) by merely adding a solution of the aforementioned compound (B) to a mixture containing a bead of a mercapto group-containing uronic acid-based polymer, water and the like and standing the obtained mixture. Examples of the solvent for the aforementioned solution include water, dimethylformamide (DMF), dimethyl sulfoxide (DMSO), acetonitrile and the like. The concentration of the solution of the aforementioned compound (B) is, for example, 1 μmM - 100 mM. The standing time of the mixture after addition of the aforementioned solution of compound (B) is, for example, 10 min - 5 hr, and the temperature thereof is, for example, 10 - 100°C.

The reaction between compound (B) wherein $R^{b1}$ is a group other than a 1-maleimidyl group or organic group containing a 1-maleimidyl group and a mercapto group-containing uronic acid-based polymer can be performed by adding a solution of compound (B) to a mixture of a mercapto group-containing uronic acid-based polymer bead, water and the like. Examples of the solvent for the aforementioned solution include water, dimethylformamide (DMF), dimethyl sulfoxide (DMSO), acetonitrile and the like. Where necessary, a dilute solution of sodium hydroxide may be added to the reaction system to adjust the pH thereof to 6 - 7. The reaction time is 10 min - 5 hr and the temperature thereof is 10 - 100°C.

In the bead of the present invention, the proportion of the uronic acid unit having a mercapto group in the total constitutional units of the mercapto group-containing uronic acid-based polymer is preferably 0.1 - 50 mol%, more preferably 0.1 - 30 mol%, further preferably 1 - 10 mol%. This proportion can be calculated from a ratio of a peak area of proton on a carbon atom bonded to a mercapto group (-SH) and a peak area of proton on the carbon skeleton of uronic acid, which is obtained when a sample is measured by a nuclear magnetic resonance apparatus.

In the bead of the present invention, a disulfide bond (-S-S-) is formed from mercapto groups (-SH) by natural oxidation. Due to this disulfide bond, the bead of the present invention shows superior durability. In the bead of the present invention, a proportion of the mercapto group forming the disulfide bond in the total mercapto groups is preferably 10 - 100 mol%, more preferably 50 - 100 mol%, further preferably 70 - 100 mol%. This proportion can be calculated by measuring the amount of mercapto group immediately after bead production when the disulfide bond is considered to be nil and the amount of mercapto group after progress of a given time from the production of a bead considered to be formed by the disulfide bond by the Elleman method and comparing them.

The mercapto group-containing uronic acid-based polymer preferably contains a carboxy group in addition to the mercapto group. This carboxy group may be in a free acid (-COOH) form or an anion (-COO-) form. The amount of the carboxy group in the mercapto group-containing uronic acid-based polymer is preferably 80 - 100 mol, more preferably 90 - 100 mol, further preferably 95 - 100 mol, relative to 100 moles of the total constitutional units of the mercapto group-containing uronic acid-based polymer. This amount can be measured by a ratio of a peak area of proton on a carbon

atom bonded to a mercapto group (-SH) and a peak area of proton on the carbon skeleton of uronic acid, which is obtained when a sample is measured by a nuclear magnetic resonance apparatus.

The number average molecular weight of the mercapto group-containing uronic acid-based polymer is preferably 25,000 - 500,000, more preferably 25,000 - 300,000, further preferably 25,000 - 100,000. The number average molecular weight of a polymer crosslinked by forming a disulfide bond cannot be measured. Therefore, this number average molecular weight is a value of a polymer not forming a disulfide bond. This number average molecular weight can be measured by gel permeation chromatography (GPC).

The bead of the present invention can be produced by adding a mercapto group-containing uronic acid-based polymer to an aqueous solution containing a divalent metal ion. The thus-produced bead of the present invention contains a divalent metal ion.

Examples of the divalent metal ion include alkaline earth metal ion and the like. Among these, calcium ion, barium ion and strontium ion are preferable, and calcium ion is more preferable. Only one kind or two or more kinds of the divalent metal ion may be used. The amount of the divalent metal ion in the bead of the present invention is preferably 1 - 300 mmol, more preferably 10 - 200 mmol, further preferably 20 - 100 mmol, per 1 L of the bead. This amount can be measured by atomic absorption analysis.

Examples of the aqueous solution containing a divalent metal ion include aqueous calcium chloride solution, aqueous barium chloride solution, aqueous strontium chloride solution and the like. Among these, aqueous calcium chloride solution and aqueous barium chloride solution are preferable, and aqueous calcium chloride solution is more preferable. The concentration of the divalent metal ion in the aqueous solution containing a divalent metal ion is preferably 10 - 200 mM, more preferably 20 - 100 mM.

When the mercapto group-containing uronic acid-based polymer has a carboxy group, it is preferable to prepare an aqueous solution of the mercapto group-containing uronic acid-based polymer by converting the carboxy group to an anion form with an alkali metal hydroxide. The aforementioned alkali metal hydroxide is preferably sodium hydroxide.

It is preferable to form a bead by adding an aqueous solution of the mercapto group-containing uronic acid-based polymer to an aqueous solution containing the aforementioned divalent metal ion. The concentration of the mercapto group-containing uronic acid-based polymer in the aqueous solution to be added is preferably not less than 0.5 w/v%, more preferably not less than 1 w/v%, further preferably not less than 2 w/v%, preferably not more than 30 w/v%, more preferably not more than 20 w/v%, further preferably not more than 10 w/v%.

It is preferable to use a syringe when adding an aqueous solution containing the mercapto group-containing uronic acid-based polymer. The inner diameter of the needle of the syringe is preferably 0.14 - 2.27 mm, more preferably 0.14 - 0.52 mm.

The temperature when adding an aqueous solution of the mercapto group-containing uronic acid-based polymer (i.e., temperature of the aforementioned aqueous solution and temperature of aqueous solution containing divalent metal ion) is preferably 4 - 37°C, more preferably 10 - 30°C.

The bead of the present invention may further contain a monovalent metal ion. Examples of the monovalent metal ion include sodium ion, potassium ion and the like.

The average particle diameter of the bead of the present invention is preferably 0.01 - 20 mm, more preferably 0.1 - 5 mm, further preferably 0.2 - 2 mm. In the present invention, "average particle diameter of bead" means, unless particularly described, an average maximum diameter of randomly selected 5 beads. This average particle diameter can be measured using a microscope and digital camera. To be specific, the average particle diameter of the bead can be calculated by taking a microphotograph of the beads at 4x magnification using a microscope and a digital camera, randomly selecting 5 beads, and calculating the maximum diameters of the 5 selected beads in the photograph with the software attached to the digital camera.

The bead of the present invention preferably contains water. The water amount of the bead of the present invention is preferably not less than 80 wt%, more preferably not less than 90 wt%, further preferably not less than 95 wt%, preferably not more than 99.5 wt%, more preferably not more than 99 wt%, further preferably not more than 98 wt%, particularly preferably not more than 97 wt%. This water content can be calculated by comparing the bead weight before and after drying. To be specific, 50 beads are randomly selected, surface moisture is removed, and the total weight thereof before drying is measured. Then, these are dried in a thermostatic dryer at 100°C for 3 hr and the total weight thereof after drying is measured. The water amount of one bead can be calculated by comparing the obtained total bead weights before and after drying.

The amount of the mercapto group-containing uronic acid-based polymer in the bead of the present invention is preferably not less than 0.5 wt%, more preferably not less than 1 wt%, further preferably not less than 2 wt%, particularly preferably not less than 3 wt%, preferably not more than 20 wt%, more preferably not more than 10 wt%, further preferably not more than 5 wt%.

The bead of the present invention enclosing a cell or microorganism in the inside can be produced, for example, by adding a suspension containing a cell or microorganism and the mercapto group-containing uronic acid-based polymer to an aqueous solution containing a divalent metal ion. The explanation of the divalent metal ion (kind and concentration

of ion) is as mentioned above.

When the mercapto group-containing uronic acid-based polymer has a carboxy group, the aforementioned polymer is preferably a salt with an alkali metal (particularly salt with alkali metal hydroxide). The aforementioned alkali metal is preferably sodium and the aforementioned alkali metal hydroxide is preferably sodium hydroxide.

The concentration of the aforementioned polymer in a suspension containing a cell or microorganism and the mercapto group-containing uronic acid-based polymer is preferably 1 - 30 w/v%, more preferably 2 - 10 w/v%. When the aforementioned suspension contains a cell, the cell mass is preferably $1.0\times10$ - $1.0\times10^9$ cells/mL, more preferably $1.0\times10^2$ - $1.0\times10^7$ cells/mL. The cell mass can be measured by MTT assay using 3-(4,5-dimethylthiazol-2-yl)-2,5-diphenyltetrazolium bromide (MTT). When the aforementioned suspension contains a microorganism, the microbial biomass is preferably $1.0\times10$ - $1.0\times10^9$ microorganisms/mL, more preferably $1.0\times10^2$ - $1.0\times10^7$ microorganisms/mL. The microbial biomass can be measured by the surface plate method.

It is preferable to use a syringe when adding a suspension containing a cell or microorganism and the mercapto group-containing uronic acid-based polymer. The inner diameter of the needle of the syringe is preferably 0.14 - 2.27 mm, more preferably 0.14 - 0.52 mm.

The temperature when adding a suspension containing a cell or microorganism and the mercapto group-containing uronic acid-based polymer (i.e., temperature of the aforementioned suspension and temperature of the aqueous solution containing a divalent metal ion) is preferably 4 - 37°C, more preferably 10 - 37°C.

The average particle diameter of the cell or microorganism-enclosing bead of the present invention is preferably 0.01 - 20 mm, more preferably 0.1 - 5 mm, further preferably 0.2 - 2 mm. This average particle diameter can be measured using a microscope and digital camera, as mentioned above.

The cell mass of the cell-enclosing bead of the present invention is preferably $1.0\times10$ - $1.0\times10^9$ cells/mL, more preferably $1.0\times10^2$ - $1.0\times10^7$ cells/mL. The cell mass can be measured by MTT assay. The microbial biomass of the microorganism-enclosing bead of the present invention is preferably $1.0\times10$ - $1.0\times10^9$ microorganisms/mL, more preferably $1.0\times10^2$ - $1.0\times10^7$ microorganisms/mL. The microbial biomass can be measured by the surface plate method.

The bead of the present invention optionally further has at least one outer layer (e.g., two outer layers). Examples of such bead include one further having the first layer and the second layer as outer layers, in which the first layer is formed on the bead of the present invention and the second layer is formed on the first layer. In the following, the bead of the present invention having the first layer and the second layer is sometimes referred to as the core-shell type bead of the present invention.

The explanation of the bead (core) of the core-shell type bead of the present invention is as mentioned above unless particularly described. In a polymer containing uronic acid units having mercapto groups contained in the bead, the aforementioned uronic acid is at least one selected from the group consisting of mannuronic acid and guluronic acid, and the polymer containing uronic acid units having mercapto groups is preferably an alginic acid having mercapto groups.

The aforementioned uronic acid unit having a mercapto group is preferably one in which a uronic acid residue and a residue of compound (A1) or compound (A2) is bonded via an amide bond. The explanation of the compound (A1) and compound (A2) in the core-shell type bead of the present invention is as mentioned above unless particularly described. Of these compound (A1) and compound (A2), compound (A1) is preferable, cysteine and homocysteine are more preferable, and cysteine is further preferable.

The thickness of the first layer (one of shells) of the core-shell type bead of the present invention is preferably 0.1 - 200 $\mu$m, more preferably 1 - 100 $\mu$m. The first layer is preferably formed from at least one selected from the group consisting of water-soluble chitosan and polyornithine, more preferably water-soluble chitosan or polyornithine, further preferably water-soluble chitosan. Water-soluble chitosan is advantageous in that it does not easily induce an inflammation reaction in the body compared with polyornithine.

The number average molecular weight of the water-soluble chitosan is preferably 10,000 - 1,000,000, more preferably 50,000 - 500,000. The number average molecular weight can be measured by gel permeation chromatography (GPC). The number average molecular weight of polyornithine is preferably 10,000 - 300,000, more preferably 10,000 - 100,000. This number average molecular weight can be measured by gel permeation chromatography (GPC).

Examples of the water-soluble chitosan include glycol chitosan obtained by bonding glycol to chitosan and aldonic acid-modified chitosan obtained by bonding aldonic acid to chitosan. Examples of the glycol include ethylene glycol, propylene glycol, diethylene glycol and the like. Examples of the aldonic acid include threonic acid, xylonic acid, gluconic acid and the like. The second layer is particularly preferably formed from glycol chitosan.

Ornithine constituting polyornithine may be any of L-form and D-form, and preferably L-form. Polyornithine may be in the form of a salt with an inorganic acid. The acid for forming the polyornithine salt may be any of an organic acid and an inorganic acid, preferably an inorganic acid. Examples of the inorganic acid include hydrobromic acid, hydrochloric acid and the like. Among these, hydrobromic acid is preferable. Polyornithine for forming the first layer is preferably poly-L-ornithine hydrobromide.

Water-soluble chitosan is available from, for example, Sigma-Aldrich. Polyornithine is available from, for example, Sigma-Aldrich.

The thickness of the second layer (one of shells) of the core-shell type bead of the present invention is preferably 0.1 - 200 μm, more preferably 1 - 100 μm. The second layer is preferably formed from at least one selected from the group consisting of polygalacturonic acid and polygalacturonic acid having mercapto groups (hereinafter sometimes to be referred to as "polygalacturonic acid and the like"). Polygalacturonic acid does not permit easy proliferation of immunocytes such as macrophage and the like thereon. Using this as the second layer (the outermost layer), a foreign-body reaction in the body can be inhibited.

The number average molecular weight of the polygalacturonic acid for forming the second layer is preferably 25,000 - 500,000, more preferably 25,000 - 300,000, further preferably 25,000 - 100,000. This number average molecular weight can be measured by gel permeation chromatography (GPC).

The polygalacturonic acid and the like for forming the second layer preferably contains a divalent metal ion. The polygalacturonic acid and the like can form the second layer even if they do not contain a divalent metal ion. Examples of the divalent metal ion include alkaline earth metal ion and the like. Among these, calcium ion, barium ion and strontium ion are preferable, and calcium ion is more preferable. Only one kind or two or more kinds of divalent metal ion may be used. The amount of the divalent metal ion in polygalacturonic acid and the like is preferably 1 - 300 mmol, more preferably 1 - 200 mmol, further preferably 1 - 100 mmol, per 1 L of the bead. This amount can be measured by atomic absorption analysis.

The polygalacturonic acid having mercapto groups for forming the second layer is preferably a galacturonic acid having a galacturonic acid residue and an amino acid residue having the mercapto group bonded to each other via an amide bond. Specific examples of the amino acid having a mercapto group include the aforementioned specific examples of compound (A1). Only one kind or two or more kinds of amino acid having a mercapto group may be used. The amino acid having a mercapto group is preferably at least one selected from the group consisting of cysteine and homocysteine, more preferably cysteine.

The proportion of the galacturonic acid unit having a mercapto group in the total constitutional units of the polygalacturonic acid having mercapto groups for forming the second layer is preferably 0.1 - 50 mol%, more preferably 0.1 - 30 mol%, further preferably 1 - 10 mol%. This proportion can be calculated from a ratio of a peak area of proton on a carbon atom bonded to a mercapto group (-SH) and a peak area of proton on the carbon skeleton of galacturonic acid, which is obtained when a sample is measured by a nuclear magnetic resonance apparatus.

The polygalacturonic acid and polygalacturonic acid having mercapto groups for forming the second layer is preferably a salt with an alkali metal (particularly salt with alkali metal hydroxide). The aforementioned alkali metal is preferably sodium and the aforementioned alkali metal hydroxide is preferably sodium hydroxide.

To form the second layer, at least one selected from the group consisting of polygalacturonic acid bonded to a compound represented by the formula (b):

$$\mathbf{H_2N\text{-}L^{b2}\text{-}Q^{b1}\text{-}L^{b3}\text{-}Q^{b2}\text{-}L^{b4}\text{-}Q^{b3}} \qquad \mathbf{(b)}$$

wherein $L^{b2}$ is a single bond or a $C_{1-6}$ alkylene group,
$Q^{b1}$ is a single bond, a phenylene group, or a $C_{4-8}$ cycloalkanediyl group,
$L^{b3}$ is a single bond or $*\text{-}(OCH_2CH_2)_n\text{-}**$ (wherein * shows a bonding position to $Q^{b1}$, ** shows a bonding position to $Q^{b2}$, and n is an integer of 1 - 10),
$Q^{b2}$ is a divalent triazole ring group,
$L^{b4}$ is a single bond, a $C_{1-6}$ alkylene group, or a $C_{1-6}$ alkylene-oxy group, and
$Q^{b3}$ is an optionally substituted phenyl group, an optionally substituted monovalent 5- or 6-membered heterocyclic group, or an optionally substituted $C_{4-8}$ cycloalkyl group, and polygalacturonic acid having mercapto groups bonded to compound (b) may also be used.

Compound (b) is the same as compound (B) except that $R^{b1}$-$L^{b1}$-CO- is not bonded to amino group. The explanation of the groups in compound (b) is the same as that for the groups in the aforementioned compound (B).

Compound (b) is preferably at least one selected from the group consisting of compound (b1), compound (b2) and compound (b3), which are represented by the following formulas, more preferably compound (b1).

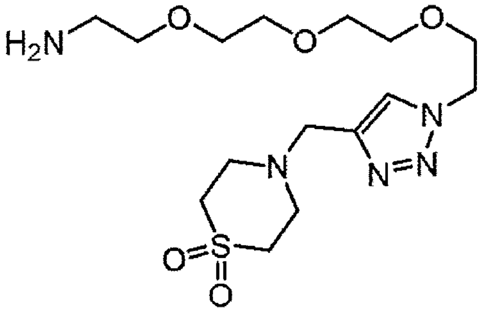

(b1)

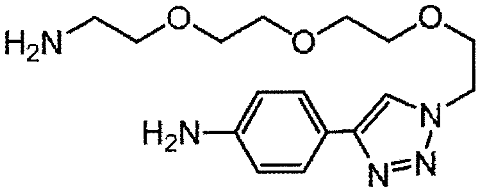

(b2)

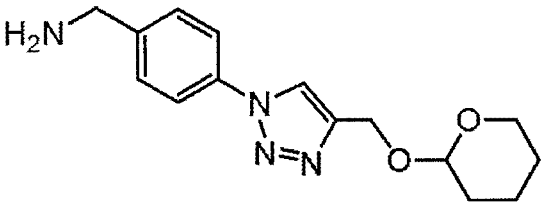

(b3)

Compound (b) is preferably a compound having an inhibitory action on a foreign-body reaction. Examples of the compound having an inhibitory action on a foreign-body reaction include the aforementioned compound (b1) to compound (b3). Compound (b) can be produced by a known method (e.g., method described in non-patent document 3). In addition, compound (b) can be bonded to polygalacturonic acid and the like by condensing the amino group of compound (b) and the carboxy group of polygalacturonic acid and the like under conditions well known to those of ordinary skill in the art.

The bead free of an outer layer of the present invention and the bead having an outer layer of the present invention (particularly core-shell type bead of the present invention) are both useful for enclosing a cell or microorganism in the inside. Explanation of the cell mass and the average particle diameter of the bead (core) and the like in the bead having an outer layer of the present invention (particularly core-shell type bead of the present invention) is the same as that for the bead free of an outer layer of the present invention.

The core-shell type bead of the present invention can be produced by, for example, as shown below. First, the bead free of an outer layer of the present invention is produced as mentioned above, the obtained bead are mixed with an aqueous solution of the polymer for forming the first layer, the bead is stood in the aforementioned aqueous solution, the bead is taken out, the first layer is formed on the bead, the obtained bead is mixed with an aqueous solution of a polymer for forming the second layer, the bead is stood in the aforementioned aqueous solution, the bead is taken out, and the second layer is formed on the first layer, whereby the core-shell type bead of the present invention can be obtained.

When polygalacturonic acid and the like are used as a polymer for forming the second layer, a bead having the first layer is mixed with an aqueous solution of polygalacturonic acid and the like, the bead is stood in the aforementioned aqueous solution, the bead is taken out, and the obtained bead is mixed with an aqueous solution containing a divalent metal ion, whereby polygalacturonic acid and the like preferably contain a divalent metal ion.

[Examples]

The present invention is explained more specifically in the following by referring to Production Examples and the like. However, the present invention is not limited by the following Production Examples and the like. The present invention can be practiced with appropriate modifications as long as they can be adapted to the above-mentioned and below-mentioned gists, all of which are encompassed in the technical scope of the present invention. The room temperature indicated below means 25°C.

The following reagents were used in the below-mentioned Production Examples and so on.
polygalacturonic acid (manufactured by Sigma-Aldrich, number average molecular weight: 25,000 - 50,000)
sodium alginate ("sodium alginate 300 - 400" manufactured by Wako Pure Chemical Industries, Ltd., viscosity of 1 w/v% aqueous solution at 25°C: 300 - 400cp)

sodium alginate ("192--09995" manufactured by Wako Pure Chemical Industries, Ltd.)
1-ethyl-3-(3-dimethylaminopropyl)carbodiimide hydrochloride ("WSCI·HCl" manufactured by WATANABE CHEMICAL INDUSTRIES, LTD.)
N-hydroxysulfosuccinimide sodium (manufactured by Tokyo Chemical Industry Co., Ltd.)
cysteine hydrochloride monohydrate (manufactured by KANTO CHEMICAL CO., INC.)
calcium chloride dihydrate (manufactured by Wako Pure Chemical Industries, Ltd.)
ethanol (manufactured by KANTO CHEMICAL CO., INC.)
trisodium citrate (manufactured by Wako Pure Chemical Industries, Ltd.)
poly-L-ornithine hydrobromide (manufactured by Sigma-Aldrich, number average molecular weight: 30,000 - 70,000)
glycol chitosan (manufactured by Sigma-Aldrich, polymer obtained by bonding ethylene glycol to chitosan with number average molecular weight of 10,000 - 300,000)

For the production of beads in the below-mentioned Examples 1, 2 and 6 - 9, and Comparative Examples 1, 2 and 5, a metal needle "SNA-30G-B" (inner diameter: 0.14 mm, hereinafter to be referred to as "30G needle") manufactured by Musashi Engineering, Inc. was used.

For the production of beads in the below-mentioned Examples 3 - 5 and Comparative Examples 3 and 4, a metal needle "SNA-28G-B" (inner diameter: 0.18 mm, hereinafter to be referred to as "28G needle") manufactured by Musashi Engineering, Inc. was used.

For the production of beads in the below-mentioned Example 10, a syringe needle 27G "NN2725R.B" (inner diameter: 0.40 mm, hereinafter to be referred to as "27G needle") manufactured by Terumo Corporation was used.

Production Example 1: Production of sodium polygalacturonate

Polygalacturonic acid (5 g) was suspended in pure water prepared by a pure water production equipment manufactured by Millipore (hereinafter to be referred to as "pure water") (24 mL). 1N Aqueous sodium hydroxide solution (24 mL) was slowly added to the obtained suspension at room temperature with stirring. After confirmation of complete dissolution of polygalacturonic acid, the obtained aqueous solution was freeze-dried to give sodium polygalacturonate as a white powder (5.6 g).

Production Example 2: Production of polygalacturonic acid having cysteine residue

By reference to the method described in non-patent document 2, polygalacturonic acid having a cysteine residue (hereinafter sometimes to be abbreviated as "cysteine-polygalacturonic acid") was produced. To be specific, sodium polygalacturonate (250 mg) obtained in Production Example 1 was dissolved in pure water (12.5 mL) at room temperature. With stirring the aqueous solution, 1-ethyl-3-(3-dimethylaminopropyl)carbodiimide hydrochloride (120 mg) and N-hydroxysulfosuccinimide sodium (54 mg) were added at room temperature. Then, 1N hydrochloric acid (200 μL) was added to the obtained mixture at room temperature to adjust the pH thereof to 5, and the mixture was further stirred at room temperature. The mixture was stirred for 1 hr and cysteine hydrochloride monohydrate (125 mg) was added to the mixture at room temperature. 1N Aqueous sodium hydroxide solution (520 μL) was added to adjust the pH thereof to 4, and the mixture was further stirred at room temperature. The mixture was stirred for 2 hr, 1N aqueous sodium hydroxide solution (200 μL) was added to adjust the pH thereof to 6, and the mixture was further stirred at room temperature. The mixture was stirred for 1 hr and ethanol (50 mL) was added slowly with a pipette at room temperature to allow for precipitation. The suspension containing the precipitate was adjusted to pH 4 with 1N hydrochloric acid (400 μL).

The suspension containing the precipitate was dispensed to two centrifugal tubes and centrifuged (2500 rpm, 3 min) to allow for complete precipitation. Using a pipette, the supernatant liquid in the centrifugal tube was discarded, water (10 mL) was newly added to the centrifugal tube and the precipitate was dissolved at room temperature. Ethanol (20 mL) was added to the obtained aqueous solution at room temperature to allow for precipitation. The suspension containing the precipitate was centrifuged (2500 rpm, 3 min) to allow for complete precipitation. This centrifugation operation was performed 3 times in total.

The precipitates in the two centrifugal tubes were dissolved in water, the obtained aqueous solution was filled in a dialysis tube (Biotech CE Tubing MWCO: 8-10kD), and dialysis was performed at room temperature using the following dialysis solution. The first dialysis was performed using a 0.15 M aqueous sodium chloride solution for 9 hr. The second dialysis was performed using a 0.15 M aqueous sodium chloride solution for 16 hr. The third dialysis was performed using 1 mM hydrochloric acid for 6 hr. After these dialyses, the aqueous solution in the dialysis tube was freeze-dried to give cysteine-polygalacturonic acid as a white powder (95 mg). The results of $^{1}H$-NMR (proton nuclear magnetic resonance) measured by the below-mentioned method are shown below.
$^{1}H$-NMR (400 MHz, $D_2O$) $\delta$ (ppm) : 2.75-3.0(m), 3.50-3.75(m), 3.75-4.0(m), 4.20-4.40(m), 4.80-5.25(m)

Production Example 3: Production of polygalacturonic acid as white powder (control synthesis)

To confirm the effect of purification in Production Example 2, the same condensation and purification operation was performed using the same materials as in Production Example 2 except that a condensing agent 1-ethyl-3-(3-dimethylaminopropyl)carbodiimide hydrochloride was not used to avoid a condensation reaction to obtain polygalacturonic acid as a white powder (106 mg). The results of $^1$H-NMR (proton nuclear magnetic resonance) measured by the below-mentioned method are shown below.
$^1$H-NMR (400 MHz, $D_2O$) $\delta$ (ppm) : 3.50-3.75(m), 3.75-4.0(m), 4.20-4.40(m), 4.80-5.25(m)

Production Example 4: Production of alginic acid having cysteine residue

In the same manner as in Production Example 2 except that sodium alginate ("sodium alginate 300 - 400" manufactured by Wako Pure Chemical Industries, Ltd.) was used instead of sodium polygalacturonate, alginic acid having a cysteine residue (hereinafter sometimes to be abbreviated as "cysteine-alginic acid") was obtained as a white powder (100 mg). The results of $^1$H-NMR (proton nuclear magnetic resonance) measured by the below-mentioned method are shown below.
$^1$H-NMR (400 MHz, $D_2O$) $\delta$ (ppm): 2.75-2.90(m), 3.25-4.30(m), 4.30-5.80(m)

Production Example 5: Production of alginic acid as white powder (control synthesis)

To confirm the effect of purification in Production Example 4, the same condensation and purification operation was performed using the same materials as in Production Example 4 except that a condensing agent 1-ethyl-3-(3-dimethylaminopropyl)carbodiimide hydrochloride was not used to avoid a condensation reaction to obtain alginic acid as a white powder (76 mg). The results of $^1$H-NMR (proton nuclear magnetic resonance) measured by the below-mentioned method are shown below.
$^1$H-NMR (400 MHz, $D_2O$) $\delta$ (ppm) : 3.25-4.30(m), 4.30-5.80(m)

Production Example 6: Production of alginic acid having cysteine residue

Sodium alginate (manufactured by Wako Pure Chemical Industries, Ltd. "192--09995") (500 mg) was dissolved in pure water (12.5 mL) at room temperature. With stirring the aqueous solution, 1-ethyl-3-(3-dimethylaminopropyl)carbodiimide hydrochloride (240 mg) and N-hydroxysulfosuccinimide sodium (108 mg) were added at room temperature. Then, 1N hydrochloric acid (400 $\mu$L) was added to the obtained mixture at room temperature to adjust the pH thereof to 5, and the mixture was further stirred at room temperature. The mixture was stirred for 1 hr and cysteine hydrochloride monohydrate (1000 mg) was added to the mixture at room temperature. 1N Aqueous sodium hydroxide solution (4.5 mL) was added to adjust the pH thereof to 4, and the mixture was further stirred at room temperature. The mixture was stirred for 2 hr, 1N aqueous sodium hydroxide solution (1200 $\mu$L) was added to adjust the pH thereof to 6, and the mixture was further stirred at room temperature. The mixture was stirred for 1 hr and 1N hydrochloric acid (600 $\mu$L) was added to adjust the pH thereof to 4. Ethanol (100 mL) was added to the mixture slowly with a pipette at room temperature to allow for precipitation.
The suspension containing the precipitate was dispensed to 4 centrifugal tubes and centrifuged (2500 rpm, 3 min) to allow for complete precipitation. The supernatant liquid in the centrifugal tube was discarded, water (10 mL) was newly added to the centrifugal tube and the precipitate was dissolved by shaking well. Ethanol (20 mL) was added to the obtained aqueous solution at room temperature to allow for precipitation. The suspension containing the precipitate was centrifuged (2500 rpm, 3 min) to allow for complete precipitation. This centrifugation operation was performed 3 times in total.
The precipitates in the 4 centrifugal tubes were dissolved in water, the obtained aqueous solution was filled in two dialysis tubes (Biotech CE Tubing MWCO: 8-10kD), and dialysis was performed at room temperature using the following dialysis solution. The first dialysis was performed using a 0.15 M aqueous sodium chloride solution and 1 mM hydrochloric acid for 8 hr. The second dialysis was performed using a 0.15 M aqueous sodium chloride solution and 1 mM hydrochloric acid for 17 hr. The third dialysis was performed using 1 mM hydrochloric acid for 6 hr. After these dialyses, the aqueous solutions in the dialysis tubes were freeze-dried to give cysteine-alginic acid as a white powder (304 mg). The results of $^1$H-NMR measured by the below-mentioned method are shown below.
$^1$H-NMR (400 MHz, $D_2O$) $\delta$ (ppm): 2.75-2.90(m), 3.25-4.30(m), 4.30-5.80(m)

Production Example 7: Production of compound (b1)

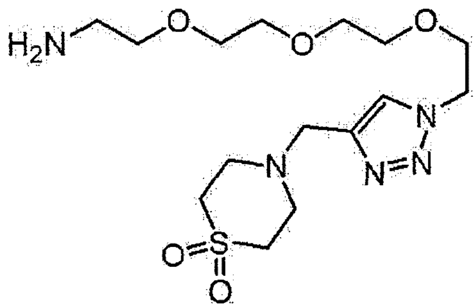

(b1)

Compound (b1) (i.e., 2-[2-[2-[2-[4-[(1,1-dioxo-1,4-thiazinan-4-yl)methyl]triazol-1-yl]ethoxy]ethoxy]ethoxy]ethaneamine) was produced as described in the experiment of non-patent document 3 (Nature Biotechnology, Vol 34, No. 3 (2016), 345-352).

Production Example 8: Production of compound (B1)

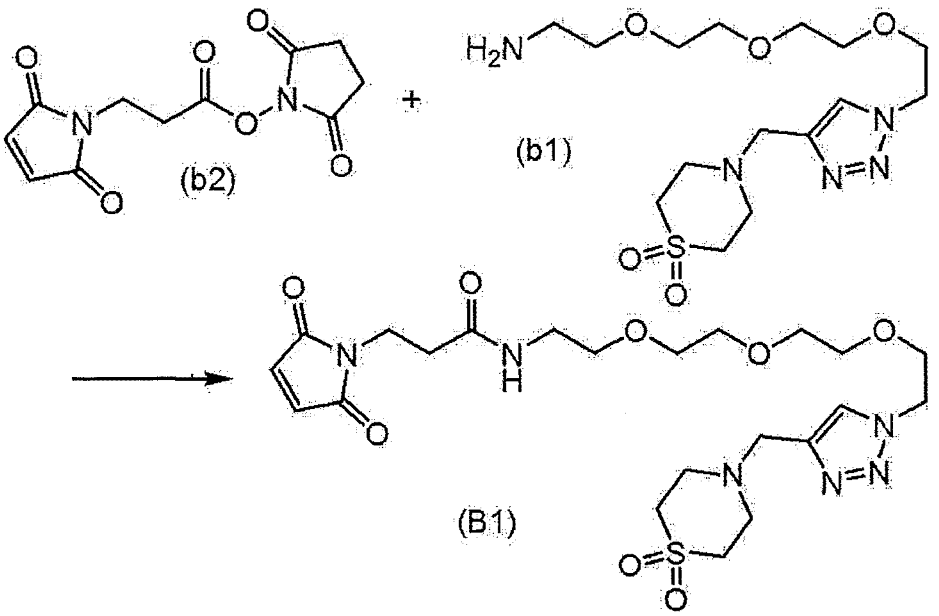

(b2) (b1) (B1)

To a solution of compound (b1) (0.965 g, 2.46 mmol) in dimethylformamide (DMF) (20 mL) were added N,N-diisopropylethylamine (0.637 mL, 3.70 mmol) and compound (b2) (i.e., 3-maleimidopropionic acid N-hydroxysuccinimide ester) (0.655 g, 2.46 mmol), and the mixture was stirred at room temperature overnight. To the reaction mixture was added 0.1N hydrochloric acid (20 mL) and the mixture was extracted with ethyl acetate. The solvent was evaporated and the obtained residue was purified by high performance liquid chromatography (water-acetonitrile, each containing 0.1% by volume trifluoroacetic acid) to give compound (B1) (i.e., 2-[3-[2-[2-[2-[2-[4-[(1,1-dioxo-1,4-thiazinan-4-yl)methyl]triazol-1-yl]ethoxy]ethoxy]ethoxy]ethylamino]but-3-enyl]cyclopenta-4-en-1,3-dione) (0.398 g, 0.733 mmol, yield 30%). The results of MS (mass spectrum) and $^1$H-NMR measured by the below-mentioned methods are described below.
MS(ESI) m/z 543$(M+H)^+$
$^1$H NMR (400 MHz, DMSO-$d_6$) δ 8.00(s, 1H), 7.00(s, 1H), 4.51(t, J=5.3Hz, 1H), 3.82(t, J=5.3Hz, 1H), 3.77(s, 1H), 3.60(dd, J=7.9, 6.6Hz, 1H), 3.54-3.50(m, 1H), 3.50-3.46(m, 3H), 3.41-3.30(m, 4H), 3.20-3.07(m, 3H), 2.96-2.87(m, 2H), 2.72(s, 1H), 2.67-2.43(m, 12H), 2.37(s, 1H), 2.33(m, 1H).

Production Example 9: Production of alginic acid bonded to compound (b1)

Alginic acid bonded to compound (b1) was produced under the conditions described in the experiment of non-patent document 3 (Nature Biotechnology, Vol 34, No. 3 (2016), 345-352). The aforementioned alginic acid was purified by the operation described in Production Example 2. The results of $^1$H-NMR measured by the below-mentioned method

are shown below. $^1$H-NMR (400 MHz, $D_2O$) δ (ppm): 8.06-7.92(m), 5.29-4.31(m), 4.22-3.27(m), 3.26-3.13(m), 3.07-2.91(m)

Production Example 10: Production of polygalacturonic acid bonded to compound (b1)

Sodium polygalacturonate (250 mg) was dissolved in 0.1 M MES buffer (50 mL, pH:6.0) at room temperature. While stirring the obtained aqueous solution at room temperature, 1-ethyl-3-(3-dimethylaminopropyl)carbodiimide hydrochloride (241 mg) and N-hydroxysulfosuccinimide sodium (137 mg) were added and the mixture was further stirred for 1 hr to give solution 1.

Then, compound (b1) (125 mg) was dissolved in a mixed solvent of pure water (1.5 mL) and acetonitrile (1.5 mL) to prepare solution 2. Solution 2 was added to the aforementioned solution 1 and the obtained mixture was stirred at room temperature for 20 hr. After stirring, ethanol (80 mL) was added slowly to the mixture with a pipette at room temperature to allow for precipitation to give a suspension.

The obtained suspension was dispensed to two centrifugal tubes and centrifuged (2500 rpm, 3 min) to allow for complete precipitation. Using a pipette, the supernatant liquid in the centrifugal tube was discarded, water (10 mL) was newly added to the centrifugal tube and the precipitate was dissolved at room temperature. The obtained aqueous solution was filled in a dialysis tube (Biotech CE Tubing MWCO: 8-10kD) and dialysis was performed 3 times at room temperature using a dialysis solution of a mixture of 0.15 M aqueous sodium chloride solution and 1 mM hydrochloric acid. Respective dialysis times were 4 hr, 18 hr and 6 hr. Then using 1 mM hydrochloric acid as a dialysis solution, dialysis was performed for 7 hr. After these dialyses, the aqueous solution in the dialysis tube was freeze-dried to give polygalacturonic acid bonded to compound (b1) as a white powder (260 mg) .

The results of $^1$H-NMR measured by the below-mentioned method are shown below.
$^1$H-NMR (400 MHz, $D_2O$) δ (ppm): 8.10-7.90(m), 5.10-4.90(m), 4.90-4.40(m), 4.40-4.10(m), 4.10-3.80(m), 3.80-3.65(m), 3.65-3.45(m), 3.26-3.20(m), 3.05-2.90(m)

$^1$H-NMR measurement

Each white powder (20 mg) obtained in Production Examples 2 - 6, compound (B1) (20 mg) obtained in Production Example 8, compound (b1)-bonded alginic acid (20 mg) obtained in Production Example 9 or compound (b1)-bonded polygalacturonic acid bonded obtained in Production Example 10 was suspended in $D_2O$ (1 mL). 1N NaOD solution (solvent: $D_2O$) (40 μL) was added to the obtained suspension and the white powder was dissolved. $^1$H-NMR of the obtained solution was measured using 400 MHz nuclear magnetic resonance analysis device manufactured by Bruker.

A peak of cysteine was not observed in $^1$H-NMR of the white powders of polygalacturonic acid and alginic acid obtained in Production Examples 3 and 5. Therefrom it was confirmed that the material (free cysteine) used for producing cysteine-polygalacturonic acid or cysteine-alginic acid can be completely removed by the purification operation performed in Production Examples 2 and 4.

A peak of cysteine was observed in δ 2.75 - 3.00 in $^1$H-NMR of the white powder obtained in Production Example 2. Complete removal of free cysteine by purification was confirmed from the $^1$H-NMR results of Production Example 3 (control synthesis). Thus, the aforementioned peak is derived from the cysteine residue covalently bonded to the galacturonic acid. These results confirm that the white powder obtained in Production Example 2 was polygalacturonic acid having a cysteine residue.

From the results of $^1$H-NMR, the proportion of the galacturonic acid unit having a mercapto group in the total constitutional units of cysteine-polygalacturonic acid was calculated to be 4 mol %. From this proportion and the number average molecular weight of polygalacturonic acid as the material, the number average molecular weight of cysteine-polygalacturonic acid was calculated to be 25,600 - 51,300.

A peak of cysteine was observed in δ 2.70 - 2.90 in $^1$H-NMR of the white powder obtained in Production Example 4. Complete removal of free cysteine by purification was confirmed from the $^1$H-NMR results of Production Example 5 (control synthesis). Thus, the aforementioned peak is derived from the cysteine residue covalently bonded to the alginic acid. These results confirm that the white powder obtained in Production Example 4 was alginic acid having a cysteine residue.

MS measurement

A solution of compound (B1) obtained in Production Example 8 in dimethyl sulfoxide (DMSO) (concentration: 100 μM) was prepared. To the obtained solution (10 μL) was added acetonitrile (330 μL) to prepare a diluted solution. Using the obtained diluted solution and ACQUITY UPLC/SQD system (manufactured by WATERS), MS of compound (B1) was measured.

Comparative Example 1: Production of alginate bead

Sodium alginate was dissolved in pure water to prepare a 2 w/v% aqueous sodium alginate solution. The obtained 2 w/v% aqueous sodium alginate solution was placed in a 5 mL syringe. A 30G needle was set to the syringe and droplets of the aqueous sodium alginate solution were added dropwise to 100 mM aqueous calcium chloride solution at room temperature to produce alginate beads.

Comparative Example 2: Production of polygalacturonate bead

Pure water (20 mL) was added to polygalacturonic acid (1.0 g) at room temperature to prepare a suspension. 1N Aqueous sodium hydroxide solution (4.7 mL) was slowly added at room temperature to the obtained suspension with stirring to prepare a transparent aqueous sodium polygalacturonate solution. The obtained aqueous solution was placed in a 5 mL syringe. A 30G needle was set to the syringe and droplets of the aqueous sodium polygalacturonate solution were added dropwise to 100 mM aqueous calcium chloride solution (25 mL) at room temperature to produce polygalacturonate beads.

Example 1: Production of cysteine-polygalacturonate bead

Cysteine-polygalacturonic acid (40 mg) was suspended in pure water (1 mL) at room temperature. 1N Aqueous sodium hydroxide solution (150 μL) was added to the obtained suspension, and the mixture was stirred at room temperature until a transparent aqueous solution was obtained to prepare an aqueous sodium cysteine-polygalacturonate solution. A 30G needle was set to 1 mL syringe and droplets of the aqueous sodium cysteine-polygalacturonate solution were slowly added dropwise to 100 mM aqueous calcium chloride solution at room temperature to produce cysteine-polygalacturonate beads.

An average particle diameter of the obtained cysteine-polygalacturonate bead was 1.6 mm. The average particle diameter was measured by the below-mentioned microscope and a digital camera. To be specific, using the microscope and a digital camera (magnification: x4), microphotographs of the beads were taken, 5 beads were randomly selected, the maximum diameters in the photograph of the selected 5 beads were calculated with the software attached to the digital camera, and the average particle diameter of the beads (average maximum diameter of 5 beads) was calculated.

Example 2: Production of cysteine-alginate bead

Cysteine-alginic acid (20 mg) was suspended in pure water (1 mL) at room temperature. 1N Aqueous sodium hydroxide solution (150 μL) was added to the obtained suspension, and the mixture was stirred at room temperature until a transparent aqueous solution was obtained to prepare an aqueous sodium cysteine-alginate solution. A 30G needle was set to 1 mL syringe and droplets of the aqueous sodium cysteine-alginate solution were added dropwise to 100 mM aqueous calcium chloride solution at room temperature to produce sodium cysteine-alginate beads. The average particle diameter of the cysteine-alginate bead measured in the same manner as in Example 1 was 1.7 mm.

Experimental Example 1: Measurement of mercapto group (-SH) concentration by Elleman method

100 mM Aqueous calcium chloride solution was added by 160 μL to a 96-well plate. 4 w/v% Aqueous sodium cysteine-polygalacturonate solution or 4w/v% aqueous sodium polygalacturonate solution was added by 20 μL to each well to produce one bead in each well. Immediately after (standing for 0 hr) bead production at room temperature and after standing for 24 hr from bead production, PBS solution for SH group measurement [mixed solution of Dulbecco's phosphate buffered saline (D-PBS(-)) (15 mL), pure water (25 mL) and 1N NaOH aqueous solution (50 μL)] (160 μL) and a solution (20 μL) obtained by dissolving 5,5'-dithiobis(2-nitrobenzoic acid) (DTNB) (39.6 mg) in Dulbecco's phosphate buffered saline (D-PBS(-)) (10 mL) were added to each well, and the mixture was further stood in a dark place for 1 hr. Thereafter, the supernatant (50 μL) was recovered from each well, diluted with pure water (150 μL), and the absorbance was determined ($\lambda_{max}$=412 nm) using microplate reader Benchmark Plus (BIO-RAD). The mercapto group concentration was calculated using an analytical curve obtained by measuring various concentrations of cysteine by a similar method. In this Experimental Example, mercapto group concentration of 8 wells was measured and an average thereof was determined.

In the well in which a bead was produced from 4 w/v% aqueous sodium polygalacturonate solution and aqueous calcium chloride solution, the mercapto group was not detected at all both at stand 0 and after 24 hr.

In the well in which a bead was produced from 4 w/v% aqueous sodium cysteine-polygalacturonate solution and aqueous calcium chloride solution, the mercapto group concentration was 0.58 μM at stand 0 hr. On the other hand, after standing for 24 hr, the mercapto group concentration decreased to 0.17 μM. Therefrom it was clarified that the

mercapto groups (-SH) changed to a disulfide bond (-S-S-) and the polymer chain was crosslinked for cysteine-polygalacturonic acid. It is considered that a disulfide bond is not formed at stand 0 hr. Thus, the proportion of the mercapto group forming the disulfide bond in the total mercapto groups is calculated to be 100x(0.58-0.17)/0.58 = 70.6 mol % in the cysteine-polygalacturonic acid bead after standing for 24 hr.

Experimental Example 2: Durability evaluation under low calcium ion ($Ca^{2+}$) conditions

For the production of an alginate bead and the like, a divalent metal ion, particularly $Ca^2$, is often used. On the other hand, the $Ca^{2+}$ concentration in the body is about 2 mM at maximum. Thus, when alginate bead containing $Ca^{2+}$ and the like are transplanted into a living organism, the $Ca^{2+}$ concentration in the bead is considered to decrease to 2 mM due to equilibrium reaction. As a result, the strength of the bead may decrease in the body. Accordingly, durability of the beads of Comparative Examples 1 and 2 and Examples 1 and 2 under low $Ca^{2+}$ conditions was evaluated.

$Ca^{2+}$ in the beads can be removed using sodium citrate since sodium citrate has the ability to bind to divalent metal ion. Thus, the obtained beads were immersed in 55 mM aqueous sodium citrate solution and the shape of the beads was observed. To be specific, 5 beads each of Comparative Examples 1 and 2 and Examples 1 and 2 were put in the wells of a 24-well plate, and 55 mM aqueous sodium citrate solution (1 mL) was added to each well such that the beads were entirely immersed in the aqueous sodium citrate solution. The 24-well plate was shaken for up to 30 hr on a rotary shaker and the shape of the beads was observed.

The following microscope (magnification: x4) and a digital camera were used for observation of the beads. The bead area in the microphotographs taken with the digital camera (hereinafter to be referred to as "bead area") was measured with the software attached to the digital camera. The area of 5 beads each of the beads obtained in Comparative Examples 1 and 2 and Examples 1 and 2 was measured and an average was calculated. The results thereof are shown in Table 1. culture microscope "CKX41" manufactured by OLYMPUS microscope digital camera "DP22" manufactured by OLYMPUS

[Table 1]

| | bead area ($mm^2$) after shaking in aqueous sodium citrate solution | | | | | |
|---|---|---|---|---|---|---|
| | 0 (before shaking) | 0.5 hr | 3 hr | 18 hr. | 24 hr | 30 hr |
| Comparative Example 1 (alginate bead) | 1.98 | dissol ved | - | - | - | - |
| Comparative Example 2 (polygalacturonate bead) | 3.52 | 4.37 | 6.11 | 6.82 | unmeasurable | - |
| Example 1 (cysteine-polygalacturonate bead) | 2.49 | 2.46 | 2.05 | 2.11 | 2.09 | 2.18 |
| Example 2 (cysteine-alginate bead) | 2.49 | 4.99 | 5.27 | 6.13 | 6.63 | 5.76 |

The alginate bead of Comparative Example 1 dissolved and completely disappeared after shaking for 30 min in 55 mM aqueous sodium citrate solution.

The polygalacturonate beads of Comparative Example 2 had a bead area of 3.52 $mm^2$ before shaking in the 55 mM aqueous sodium citrate solution. The bead area increased to 4.37 $mm^2$ after shaking for 30 min, and increased to 6.82 $mm^2$ after shaking for 18 hr. After shaking for 24 hr, the bead was further swelled and deformed and the bead area could not be measured.

The cysteine-polygalacturonate beads of Example 1 had a bead area of 2.49 $mm^2$ before shaking in the aqueous sodium citrate solution. The bead area was not more than 2.5 $mm^2$ and did not increase even after shaking for 30 min, 3 hr, 18 hr, 24 hr and 30 hr, and the bead shape remained unchanged. As is clear from these results, the cysteine-polygalacturonate bead did not swell at all under low $Ca^{2+}$ conditions.

The cysteine-alginate beads of Example 2 had a bead area of 2.49 $mm^2$ before shaking in the 55 mM aqueous sodium citrate solution. The bead area increased to 5.76 $mm^2$ after shaking for 30 hr but the bead shape was maintained. On the other hand, the alginate bead of Comparative Example 1 dissolved and disappeared after shaking for 30 min, and the polygalacturonate bead of Comparative Example 2 was swelled and deformed after shaking for 24 hr. As is clear from these results, swelling thereof can be inhibited by introducing a cysteine residue into the alginate bead.

As is clear from the comparison of Example 1 and Example 2, a bead showing still more superior durability can be obtained using a polymer containing galacturonic acid units (particularly, polygalacturonic acid) as a polymer containing uronic acid units.

Example 3: Production of cysteine-polygalacturonate bead enclosing cell in the inside and cell culture

Cells stably expressing Gα15-tarans48 LD derived from PEAKRAPID (PRG48) and maintained using DMEM/Ham's F-12 (manufactured by NACALAI TESQUE, INC.) containing 10 v/v% fetal bovine serum (manufactured by Nichirei Corporation) and 1 v/v% penicillin-streptomycin (manufactured by NACALAI TESQUE, INC.) were washed with D-PBS(-) (manufactured by NACALAI TESQUE, INC.) and the cells were recovered from 150 mm TC-treated Culture Dish (manufactured by CORNING) by using trypsin-EDTA solution [solution obtained by adding 0.25 g/L aqueous trypsin-1 mmol/L EDTA (manufactured by NACALAI TESQUE, INC.) solution to D-PBS(-) (manufactured by NACALAI TESQUE, INC.) at 20 v/v%]. The cell suspension was centrifuged (1,200 rpm, 3 min), the supernatant was removed, and the cells obtained by centrifugation were suspended in D-PBS(-) (manufactured by NACALAI TESQUE, INC.). The obtained suspension was centrifuged (1,200 rpm, 3 min), the supernatant was removed, and the cells were suspended in 4-(2-hydroxyethyl)-1-piperazineethanesulfonic acid (HEPES) buffered saline (115 mM NaCl, 5 mM KCl, 5 mM D-glucose, 15 mM HEPES, pH 7.4) at $1.0 \times 10^7$ cells/mL. The obtained cell suspension (200 μL) was added to 4 w/v% aqueous sodium cysteine-polygalacturonate solution (1.8 mL) at room temperature to give a suspension (polymer concentration: 3.6 w/v%, cell mass: $1.0 \times 10^7$ cells/mL). The obtained suspension was placed in a 1 mL syringe (Terumo) with 28G needle and added dropwise to 100 mM aqueous calcium chloride solution (25 mL) at room temperature to give beads enclosing the cells in the inside (cell mass in bead: $1.0 \times 10^7$ cells/mL).

The average particle diameter of the obtained bead enclosing the cells in the inside was 1.8 mm. This was measured in the same manner as in Example 1.

The obtained bead was washed 3 times with DMEM/Ham's F-12 (manufactured by NACALAI TESQUE, INC.), and the bead enclosing cell in the inside was placed in DMEM/Ham's F12 (manufactured by NACALAI TESQUE, INC.) and the cell was cultured (37°C, 5% $CO_2$) for 14 days.

Comparative Example 3: Production of alginate bead enclosing cell in the inside and cell culture

In the same manner as in Example 3 except that 4 w/v% aqueous sodium alginate solution was used instead of 4 w/v% aqueous sodium cysteine-polygalacturonate solution, a bead enclosing cell in the inside was obtained. In the same manner as in Example 3, the cell was cultured for 14 days.

Experimental Example 3: Evaluation of survival proportion of cell by nucleus staining method

Five cell-enclosing beads of Example 3 and Comparative Example 3 after cell culture on day 1 and day 14 were placed in each well of a 48-well plate and washed twice with D-PBS(-) (manufactured by NACALAI TESQUE, INC.) (300 μL). Thereafter, to each well were added D-PBS(-) (manufactured by NACALAI TESQUE, INC.) (150 μL), fluorescein diacetate (FDA) solution (solution obtained by adding 0.5 mg/mL FDA solution (solvent: dimethyl sulfoxide (DMSO)) (10 μL) to D-PBS(-) (manufactured by NACALAI TESQUE, INC.) (5 mL)) (150 μL), and propidium iodide (PI) solution (solution obtained by adding 1.0 mg/mL aqueous PI solution (10 μL) to D-PBS(-) (manufactured by NACALAI TESQUE, INC.) (5 mL)) (150 μL), and the mixture was stood under 37°C, 5% $CO_2$ conditions for 30 min. Living cells were stained green and dead cells were stained red. After standing, each well was washed with D-PBS(-) and observed under a fluorescence microscope BZ-X700 (manufactured by KEYENCE).

The proportion of the living cells after 14 days of culture to the living cells after 1 day of culture was 95% for the cells in the cell-enclosing cysteine-polygalacturonate bead obtained in Example 3. On the other hand, the proportion for the cells in the cell-enclosing alginate bead obtained in Comparative Example 4 was 60%. It was confirmed from these results that the cells in the cell-enclosing cysteine-polygalacturonate bead could survive at least equal to the cells in the cell-enclosing alginate bead up to day 14 of culture.

Example 4: Production of cysteine-polygalacturonate bead and bead of cysteine-polygalacturonate bonded to compound (B1)

Cysteine-polygalacturonic acid (45 mg) obtained in the same manner as in Production Example 2 was suspended in pure water (2.84 mL) at room temperature. 1N Aqueous sodium hydroxide solution (160 μL) was added to the obtained suspension, and the mixture was stirred at room temperature until a transparent aqueous solution was obtained to prepare a 1.5 w/v% aqueous sodium cysteine-polygalacturonate solution. Using a 1 mL syringe with a 28G needle, the obtained 1.5 w/v% aqueous sodium cysteine-polygalacturonate solution was added by 20 μL to 100 mM aqueous calcium chloride solution (20 mL) to produce cysteine-polygalacturonate beads.

After production as mentioned above, a mixture of bead, water and the like was stood at room temperature for 24 hr, a solution (20 μL) of 100 mM compound (B1) in dimethyl sulfoxide (DMSO) was added to the mixture, and the obtained mixture was stood at room temperature for 24 hr to produce a bead of cysteine-polygalacturonate bonded to

compound (B1).

Example 5: Production of cysteine-alginate bead and bead of cysteine-alginate bonded to compound (B1)

In the same manner as in Example 4 except that 1.5 w/v% aqueous sodium cysteine-alginate solution was prepared using cysteine-alginic acid obtained in the same manner as in Production Example 6 instead of cysteine-polygalacturonic acid, a cysteine-alginate bead and a bead of cysteine-alginate bonded to compound (B1) were produced.

Experimental Example 4: Measurement of mercapto group (-SH) concentration by Elleman method

Immediately after (standing for 0 hr) bead production at room temperature, after standing for 24 hr (standing for 24 hr) from bead production, and after standing for 24 hr at room temperature from addition of compound (B1) (24 hr from addition of compound (B1)), one bead was placed in each well of a 96-well plate, PBS solution for SH group measurement [mixed solution of Dulbecco's phosphate buffered saline (D-PBS(-)) (15 mL), pure water (25 mL) and 1N NaOH aqueous solution (50 μL)] (160 μL) and a solution (20 μL) obtained by dissolving 5,5'-dithiobis(2-nitrobenzoic acid) (DTNB) (39.6 mg) in Dulbecco's phosphate buffered saline (D-PBS(-)) (10 mL) were added to each well, and the mixture was further stood in a dark place for 1 hr. Thereafter, the supernatant (50 μL) was recovered from each well, diluted with pure water (150 μL), and the absorbance was determined ($\lambda_{max}$=412 nm) using microplate reader Benchmark Plus (BIO-RAD). The mercapto group concentration was calculated using an analytical curve obtained by measuring various concentrations of cysteine by a similar method. In this Experimental Example, the mercapto group concentration of 4 wells was measured and an average thereof was determined. The results thereof are shown in Tables 2 and 3.

[Table 2]

| cysteine-polygalacturonate bead | | | |
|---|---|---|---|
| | standing 0 hr | standing 24 hr | 24 hr from addition of compound (B1) |
| mercapto group concentration (μM) | 0.667 | 0.304 | 0.161 |
| standard deviation (μM) | 0.112 | 0.0562 | 0.109 |

[Table 3]

| cysteine-alginate bead | | | |
|---|---|---|---|
| | standing 0 hr | standing 24 hr | 24 hr from addition of compound (B1) |
| mercapto group concentration (μM) | 0.114 | 0.0699 | 0.0308 |
| standard deviation (μM) | 0.0135 | 0.0152 | 0.0156 |

Both cysteine-polygalacturonate bead and cysteine-alginate bead showed a decrease in the mercapto group concentration 24 hr after production of the bead. The decrease indicates that the mercapto groups (-SH) in the bead changed to a disulfide bond (-S-S-).

The bead after 24 hr from the addition of compound (B1) to the mixture containing cysteine-polygalacturonate bead or cysteine-alginate bead showed a further decrease in the mercapto group concentration. The decrease shows that a functional group capable of bonding to a mercapto group in compound (B1) (i.e., 1-maleimidyl group) and mercapto group in the bead reacted and a bead bonded to compound (B1) was obtained.

Comparative Example 4: Production of bead of alginate bonded to compound (b1)

Compound (b1)-bonded alginic acid (15 mg) was suspended in pure water (1 mL) at room temperature. 1N Aqueous sodium hydroxide solution (80 μL) was added to the obtained suspension, and the mixture was stirred at room temperature until a transparent aqueous solution was obtained to prepare an aqueous solution of sodium alginate bonded to compound (b1). A 28G needle was set to 1 mL syringe and droplets of the aqueous solution of sodium alginate bonded to compound (b1) were added dropwise to 100 mM aqueous calcium chloride solution at room temperature to produce beads of alginate bonded to compound (b1) .

Experimental Example 5: Durability evaluation in physiological saline

An alginate bead produced in the same manner as in Comparative Example 1 except that 1.5 w/v% aqueous sodium alginate solution was prepared, a bead of alginate bonded to compound (b1) and produced in Comparative Example 4, and a bead of cysteine-alginate bonded to compound (B1) and produced in Example 5 were stood in physiological saline (manufactured by Otsuka Pharmaceutical Co., Ltd.) at 4°C overnight. In the same manner as in Experimental Example 2 except that the area of 3 beads was measured, average bead areas after standing for 0 hr and 20 hr were determined. The results thereof are shown in Table 4.

[Table 4]

| | alginate bead | | bead of alginate bonded to compound (b1) | | bead of cysteine-alginate bonded to compound (B1) | |
|---|---|---|---|---|---|---|
| | (1) | (2) | (1) | (2) | (1) | (2) |
| bead area ($mm^2$) | 3.25 | 5.87 | 2.53 | 6.62 | 2.30 | 3.08 |
| standard deviation ($mm^2$) | 0.523 | 0.428 | 0.232 | 0.272 | 0.100 | 0.407 |
| (note)<br>(1) = standing for 0 hr<br>(2) = after standing for 20 hr | | | | | | |

As shown in Table 4, the bead of cysteine-alginate bonded to compound (B1) has a smaller bead area after standing for 20 hr and shows superior durability compared to the alginate bead and the bead of alginate bonded to compound (b1).

Comparative Example 5: Production of alginate (second layer)/polyornithine salt (first layer)/alginate bead

Sodium alginate was dissolved in pure water to prepare a 1.5 w/v% aqueous sodium alginate solution. The obtained 1.5 w/v% aqueous sodium alginate solution was placed in a 1 mL syringe. A 30G needle was set to the syringe and droplets of the aqueous sodium alginate solution were added dropwise to 100 mM aqueous calcium chloride solution at room temperature to produce alginate beads.

After 10 min from dropwise addition of the aqueous sodium alginate solution, the supernatant liquid of the suspension containing the beads was discarded and the obtained beads were washed 3 times with pure water (5 mL). Then, an aqueous solution (5 mL) of 0.1 w/v% poly-L-ornithine hydrobromide was added to the washed beads. After standing for 10 min from the addition, the supernatant liquid of the suspension containing the beads was discarded, and the obtained polyornithine salt (first layer)/alginate beads were washed 3 times with pure water (5 mL).

Successively, 0.25 w/v% aqueous sodium alginate solution (5 mL) was added to the washed beads. After standing for 10 min from the addition, the supernatant liquid of the suspension containing the beads was discarded and the obtained beads were washed 3 times with pure water (5 mL). Then, 100 mM aqueous calcium chloride solution (5 mL) was added to the obtained beads to give alginate (second layer)/polyornithine salt (first layer)/alginate bead.

Example 6: Production of polygalacturonate (second layer)/polyornithine salt (first layer)/cysteine-alginate bead

Cysteine-alginic acid (90 mg) was suspended in pure water (6 mL) at room temperature. 1N Aqueous sodium hydroxide solution (140 μL) was added to the obtained suspension to prepare 1.5 w/v% aqueous sodium cysteine-alginate solution with pH 6. The obtained aqueous solution was placed in a 1 mL syringe. A 30G needle was set to the syringe and droplets of the aqueous sodium cysteine-alginate solution were added dropwise to 100 mM aqueous calcium chloride solution (25 mL) at room temperature to produce cysteine-alginate beads.

After 10 min from dropwise addition of the aqueous sodium cysteine-alginate solution, the supernatant liquid of the suspension containing the beads was discarded and the obtained beads were washed 3 times with pure water (5 mL). Then, an aqueous solution (5 mL) of 0.1 w/v% poly-L-ornithine hydrobromide was added to the washed beads. After standing for 10 min from the addition, the supernatant liquid of the suspension containing the beads was discarded, and the obtained polyornithine salt (first layer)/cysteine-alginate beads were washed 3 times with pure water (5 mL).

Successively, 0.1 w/v% aqueous sodium polygalacturonate solution (5 mL) was added to the washed beads. This aqueous sodium polygalacturonate solution was prepared by suspending polygalacturonic acid (10 mg) in pure water (10 mL) and adding 1N aqueous sodium hydroxide solution (25 μL) to the obtained suspension.

After standing for 10 min from the addition of the aqueous sodium polygalacturonate solution, the supernatant liquid of the suspension containing the beads was discarded and the obtained beads were washed 3 times with pure water (5 mL). A 100 mM aqueous calcium chloride solution (5 mL) was added to the obtained beads to give polygalacturonate (second layer)/polyornithine salt (first layer)/cysteine-alginate beads.

Example 7: Production of polygalacturonate (second layer)/glycol chitosan (first layer)/cysteine-alginate bead

Cysteine-alginic acid (90 mg) was suspended in pure water (6 mL) at room temperature. 1N Aqueous sodium hydroxide solution (140 μL) was added to the obtained suspension to prepare 1.5 w/v% aqueous sodium cysteine-alginate solution with pH 6. The obtained aqueous solution was placed in a 1 mL syringe. A 30G needle was set to the syringe and droplets of the aqueous sodium cysteine-alginate solution were added dropwise to 100 mM aqueous calcium chloride solution at room temperature to produce cysteine-alginate beads.

After 10 min from dropwise addition of the aqueous sodium cysteine-alginate solution, the supernatant liquid of the suspension containing the beads was discarded and the obtained beads were washed 3 times with pure water (5 mL). Then, 0.1 w/v% aqueous glycol chitosan solution (5 mL) was added to the obtained beads. After standing for 10 min from the addition, the supernatant liquid of the suspension containing the beads was discarded, and the obtained glycol chitosan (first layer)/cysteine-alginate beads were washed 3 times with pure water (5 mL).

Successively, 0.1 w/v% aqueous sodium polygalacturonate solution (5 mL) was added to the washed beads. This aqueous sodium polygalacturonate solution was prepared by suspending polygalacturonic acid (10 mg) in pure water (10 mL) and adding 1N aqueous sodium hydroxide solution (25 μL) to the obtained suspension.

After standing for 10 min from the addition of the aqueous sodium polygalacturonate solution, the supernatant liquid of the suspension containing the beads was discarded and the obtained beads were washed 3 times with pure water (5 mL). A 100 mM aqueous calcium chloride solution (5 mL) was added to the obtained beads to give polygalacturonate (second layer)/glycol chitosan (first layer)/cysteine-alginate beads.

Example 8: Production of cysteine-polygalacturonate (second layer)/glycol chitosan (first layer)/cysteine-alginate bead

Cysteine-alginic acid (90 mg) was suspended in pure water (6 mL) at room temperature. 1N Aqueous sodium hydroxide solution (140 μL) was added to the obtained suspension to prepare 1.5 w/v% aqueous sodium cysteine-alginate solution with pH 6. The obtained 1.5 w/v% aqueous sodium cysteine-alginate solution was placed in a 1 mL syringe. A 30G needle was set to the syringe and droplets of the aqueous sodium cysteine-alginate solution were added dropwise to 100 mM aqueous calcium chloride solution (25 mL) at room temperature to produce cysteine-alginate beads.

After 10 min from dropwise addition of the aqueous sodium alginate solution, the supernatant liquid of the suspension containing the beads was discarded and the obtained beads were washed 3 times with pure water (5 mL). Then, 0.1 w/v% aqueous glycol chitosan solution (5 mL) was added to the obtained beads. After standing for 10 min from the addition, the supernatant liquid of the suspension containing the beads was discarded, and the obtained glycol chitosan (first layer)/cysteine-alginate beads were washed 3 times with pure water (5 mL).

Successively, 0.1 w/v% aqueous sodium cysteine-polygalacturonate solution (5 mL) was added to the washed beads. This aqueous sodium cysteine-polygalacturonate solution was prepared by suspending cysteine-polygalacturonic acid (10 mg) in pure water (10 mL) and adding 1N aqueous sodium hydroxide solution (25 μL) to the obtained suspension.

After standing for 10 min from the addition of the aqueous sodium cysteine-polygalacturonate solution, the supernatant liquid of the suspension containing the beads was discarded and the obtained beads were washed 3 times with pure water (5 mL). A 100 mM aqueous calcium chloride solution (5 mL) was added to the obtained beads to give cysteine-polygalacturonate (second layer)/glycol chitosan (first layer)/cysteine-alginate beads.

Example 9: Production of polygalacturonate bonded to compound (b1) (second layer)/glycol chitosan (first layer)/cysteine-alginate bead

Cysteine-alginic acid (90 mg) was suspended in pure water (6 mL) at room temperature. 1N Aqueous sodium hydroxide solution (140 μL) was added to the obtained suspension to prepare 1.5 w/v% aqueous sodium cysteine-alginate solution with pH 6. The obtained 1.5 w/v% aqueous sodium cysteine-alginate solution was placed in a 1 mL syringe. A 30G needle was set to the syringe and droplets of the aqueous sodium cysteine-alginate solution were added dropwise to 100 mM aqueous calcium chloride solution at room temperature to produce cysteine-alginate beads.

After 10 min from dropwise addition of the aqueous sodium alginate solution, the supernatant liquid of the suspension containing the beads was discarded and the obtained beads were washed 3 times with pure water (5 mL). Then, 0.1 w/v% aqueous glycol chitosan solution (5 mL) was added to the obtained beads. After standing for 10 min from the addition, the supernatant liquid of the suspension containing the beads was discarded, and the obtained glycol chitosan (first layer)/cysteine-alginate beads were washed 3 times with pure water (5 mL).

Successively, aqueous solution (5 mL) of 0.1 w/v% sodium polygalacturonate bonded to compound (b1) was added to the washed beads. This aqueous solution of sodium polygalacturonate bonded to compound (b1) was prepared by suspending compound (b1)-bonded polygalacturonic acid (10 mg) in pure water (10 mL) and adding 1N aqueous sodium hydroxide solution (50 μL) to the obtained suspension.

After standing for 10 min from the addition of the aqueous solution of sodium cysteine-polygalacturonate bonded to compound (b1), the supernatant liquid of the suspension containing the beads was discarded and the obtained beads were washed 3 times with pure water (5 mL). A 100 mM aqueous calcium chloride solution (5 mL) was added to the obtained beads to give cysteine-polygalacturonate bonded to compound (b1) (second layer)/glycol chitosan (first layer)/cysteine-alginate beads.

Experimental Example 6: Durability evaluation

Respective beads obtained in Comparative Example 5 and Examples 6 - 9 were immersed in 55 mM aqueous sodium citrate solution, 10 mM aqueous EDTA solution or physiological saline (manufactured by Otsuka Pharmaceutical Co., Ltd.) and the shape of the beads was observed. To be specific, 3 beads each were put in a 48-well plate, and 55 mM aqueous sodium citrate solution, 10 mM aqueous EDTA solution or physiological saline (each 400 μL) was added to each well such that the beads were entirely immersed in the solution. The 48-well plate was shaken for up to 180 min on a rotary shaker and the shape of the beads was observed.

The following microscope (magnification: x4) and a digital camera were used for observation of the beads. The bead area in the microphotographs taken with the digital camera (hereinafter to be referred to as "bead area") was measured with the software attached to the digital camera. The area of 3 beads each was measured and an average was calculated. culture microscope "CKX41" manufactured by OLYMPUS microscope digital camera "DP22" manufactured by OLYMPUS

The swelling rate after shaking was calculated from an average bead area before shaking and an average bead area after shaking and by the following formula:

$$\text{swelling rate (\%) after shaking} = 100 \times \text{average bead area after shaking/average bead area before shaking}$$

The results of the swelling rate after shaking for each time are shown in Tables 5 - 7.

[Table 5]

| | swelling rate (%) after shaking in aqueous sodium citrate solution | | | | | |
|---|---|---|---|---|---|---|
| | 15 min | 30 min | 60 min | 90 min | 120 min | 180 min |
| Comparative Example 5: alginate (second layer)/ polyornithine salt (first layer)/alginate bead | 154 | 211 | 245 | 262 | 264 | 274 |
| Example 6: polygalacturonate (second layer)/ polyornithine salt (first layer)/cysteine-alginate bead | 107 | 134 | 151 | 155 | 161 | 165 |
| Example 7: polygalacturonate (second layer)/glycol chitosan (first layer)/cysteine-alginate bead | 110 | 124 | 130 | 131 | 131 | 131 |
| Example 8: cysteine-polygalacturonate (second layer) /glycol chitosan (first layer)/cysteine-alginate bead | 101 | 116 | 127 | 128 | 129 | 128 |
| Example 9: polygalacturonate bonded to compound (b1) (second layer)/glycol chitosan (first layer)/ cysteine-alginate bead | 116 | 130 | 138 | 138 | 139 | 138 |

[Table 6]

| | swelling rate (%) after shaking in aqueous EDTA solution | | | | | |
|---|---|---|---|---|---|---|
| | 15 min | 30 min | 60 min | 90 min | 120 min | 180 min |
| Comparative Example 5: alginate (second layer)/polyornithine salt (first layer)/alginate bead | 125 | 227 | 328 | 335 | 349 | 353 |
| Example 6: polygalacturonate (second layer)/polyornithine salt (first layer)/cysteine-alginate bead | 106 | 133 | 159 | 168 | 171 | 172 |
| Example 7: polygalacturonate (second layer)/glycol chitosan (first layer)/cysteine-alginate bead | 126 | 136 | 148 | 153 | 151 | 153 |
| Example 8: cysteine-polygalacturonate (second layer)/glycol chitosan (first layer)/cysteine-alginate bead | 136 | 148 | 173 | 174 | 180 | 181 |
| Example 9: polygalacturonate bonded to compound (b1) (second layer)/glycol chitosan (first layer)/cysteine-alginate bead | 136 | 144 | 158 | 163 | 160 | 163 |

[Table 7]

| | swelling rate (%) after shaking in physiological saline | | | | | |
|---|---|---|---|---|---|---|
| | 15 min | 30 min | 60 min | 90 min | 120 min | 180 min |
| Comparative Example 5: alginate (second layer)/ polyornithine salt (first layer)/alginate bead | 125 | 227 | 328 | 335 | 349 | 353 |
| Example 6: polygalacturonate (second layer)/ polyornithine salt (first layer)/cysteine-alginate bead | 106 | 133 | 159 | 168 | 171 | 172 |
| Example 7: polygalacturonate (second layer)/glycol chitosan (first layer)/cysteine-alginate bead | 126 | 136 | 148 | 153 | 151 | 153 |
| Example 8: cysteine-polygalacturonate (second layer) /glycol chitosan (first layer)/cysteine-alginate bead | 136 | 148 | 173 | 174 | 180 | 181 |
| Example 9: polygalacturonate bonded to compound (b1) (second layer)/glycol chitosan (first layer)/ cysteine-alginate bead | 136 | 144 | 158 | 163 | 160 | 163 |

Example 10: Production of polygalacturonate (second layer)/glycol chitosan (first layer)/cysteine-alginate bead enclosing cell in the inside and cell culture

Human mesenchymal stem cells (hMSC) (manufactured by Lonza Japan Ltd.) maintained using DMEM medium (manufactured by Sigma Chemical Company) containing 10 v/v% fetal bovine serum (manufactured by Nichirei Corporation) and 1 v/v% penicillin-streptomycin (manufactured by NACALAI TESQUE, INC.) were washed with D-PBS(-) (manufactured by NACALAI TESQUE, INC.), the cells were recovered from 175 $mm^2$ flask (manufactured by Falcon) by using TrypLE™ Select (manufactured by Life Technologies), and an equal amount or more of a culture medium was added. The cell suspension was centrifuged (1,000 rpm, 3 min), the supernatant was removed, and the cells obtained by centrifugation were suspended in D-PBS(-) (manufactured by NACALAI TESQUE, INC.). The obtained suspension was centrifuged (1,000 rpm, 3 min), the supernatant was removed, and the cells were suspended in 4-(2-hydroxyethyl)-1-piperazineethanesulfonic acid (HEPES) buffered saline (115 mM NaCl, 5 mM KCl, 5 mM D-glucose, 15 mM HEPES, pH 7.4) at $1.0x10^7$ cells/mL. The obtained cell suspension (200 μL) was added to 1.5 w/v% aqueous sodium cysteine-alginate solution (1.8 mL) at room temperature to give a suspension (cell mass: $1.0x10^s$ cells/mL). The obtained suspension was placed in a 1 mL syringe (Terumo) with 27G needle and added dropwise to 100 mM aqueous calcium chloride solution (25 mL) at room temperature to give beads enclosing the cells in the inside (cell mass in bead: $1.0x10^5$ cells/mL).

The obtained bead was washed twice with 4-(2-hydroxyethyl)-1-piperazineethanesulfonic acid (HEPES) buffered saline (5 mL), 0.1 w/v% glycol chitosan solution was added to the washed beads and the mixture was stood for 10 min. The 0.1 w/v% aqueous glycol chitosan solution was removed, and the obtained bead was washed twice with 4-(2-hydroxyethyl)-1-piperazineethanesulfonic acid (HEPES) buffered saline (5 mL). The 0.1 w/v% glycol chitosan solution was prepared by dissolving glycol chitosan (3 mg) in 3 mL of 4-(2-hydroxyethyl)-1-piperazineethanesulfonic acid (HEPES) buffered saline (115 mM NaCl, 5 mM KCl, 5 mM D-glucose, 15 mM HEPES, pH 7.4).

A 0.15 w/v% aqueous sodium polygalacturonate solution was added to the washed beads and the mixture was stood for 5 min. The 0.15 w/v% aqueous sodium polygalacturonate solution was removed and the obtained bead was washed 3 times with DMEM/Ham's F-12 (manufactured by NACALAI TESQUE, INC.) to give polygalacturonate (second layer)/glycol chitosan (first layer)/cysteine-alginate bead enclosing human mesenchymal stem cell (hMSC) in the inside.

The beads enclosing the cells in the inside obtained as mentioned above were placed in DMEM medium (manufactured by Sigma Chemical Company) containing 10 v/v% fetal bovine serum (manufactured by Nichirei Corporation) and 1 v/v% penicillin-streptomycin (manufactured by NACALAI TESQUE, INC.) and the cells were cultured (37°C, 5% $CO_2$) for 14 days.

The proportion of the living cells after 14 days of culture to the living cells after 1 day of culture was not less than 95% for the cells in the obtained bead.

Experimental Example 7 (reference test): proliferation rate of RAW264.7 cell

A 1 w/v% aqueous sodium alginate solution (50 μL) or 1.5 w/v% aqueous sodium polygalacturonate solution (50 μL) was added to a 96-well poly-L-lysine coated plate (manufactured by IWAKI & CO., LTD.) and the mixture was stood (37°C, 5% $CO_2$) for 1 hr. A 100 mM aqueous calcium chloride solution (150 μL) was added to each well of the plate, and the mixture was stood at room temperature for 10 min. The aqueous calcium chloride solution in each well was removed and the cells were washed twice with DMEM/Ham's F-12 (manufactured by NACALAI TESQUE, INC.) (200 μL) containing 10 v/v% fetal bovine serum (manufactured by Nichirei Corporation) and 1 v/v% penicillin-streptomycin (manufactured by NACALAI TESQUE, INC.).

RAW264.7 cells (manufactured by DS Pharma Biomedical Co., Ltd.) maintained using DMEM/Ham's F-12 (manufactured by Sigma Chemical Company) containing 10 v/v% fetal bovine serum (manufactured by Nichirei Corporation) and 1 v/v% penicillin-streptomycin (manufactured by NACALAI TESQUE, INC.) were washed with D-PBS(-) (manufactured by NACALAI TESQUE, INC.), the cells were recovered from 100 mm TC-treated culture dish (manufactured by IWAKI & CO., LTD.) by using 0.25 g/L trypsin-1 mmol/L EDTA (manufactured by NACALAI TESQUE, INC.), and an equal amount or more of a medium was added. The cell suspension was centrifuged (1,200 rpm, 3 min), the supernatant was removed, and the cells obtained by centrifugation were suspended in a culture medium (manufactured by NACALAI TESQUE, INC.) at $1.0 \times 10^4$ cells/mL. The suspension was added by 100 μL to each well of a prepared 96-well plate and the cells were cultured (37°C, 5% $CO_2$) for 4 days.

Thereafter, a cell count reagent mixture (mixture of "Cell Count Reagent SF" manufactured by NACALAI TESQUE, INC. (20 μL) and culture medium (80 μL)) (100 μL) was added to each well, and the cells were cultured (37°C, 5% $CO_2$) for 3.5 hr. Thereafter, 0.1N hydrochloric acid was added to each well (10 μL/well) and the supernatant (100 μL) was recovered from each well and the absorbance was determined ($\lambda_{max}$=450 nm) using microplate reader Benchmark Plus (BIO-RAD).

The viable cell count was calculated using an analytical curve obtained by measuring various concentrations of cell suspension by a similar method. In this Experimental Example, the viable cell count of 4 wells was measured and an average thereof was determined. The proliferation rate was calculated from a viable cell count (average) when sodium alginate or sodium polygalacturonate was used and the control viable cell count (average) when none of sodium alginate and sodium polygalacturonate was used and by the following formula

proliferation rate (%) = 100 x viable cell count (average) using sodium alginate or sodium polygalacturonate/viable cell count (average) of control

The results are shown in Table 8.

[Table 8]

| | sodium alginate | sodium polygalacturonate |
|---|---|---|
| proliferation rate (%) | 69.1 | 32.1 |

The proliferation rate of RAW264.7 cells when sodium polygalacturonate was used is lower than that when sodium alginate was used. From this result, it is assumed that a core-shell type bead on which cells do not proliferate easily can be produced using sodium polygalacturonate as the second layer (outermost layer).

[Industrial Applicability]

The bead of the present invention can be used for enclosing cell or microorganism in the inside thereof. Therefore, the bead of the present invention is useful for protection and culture of cell or microorganism.

This application is based on patent application Nos. 2017-027246 and 2017-225903 filed in Japan, the contents of which are incorporated in full herein.

## Claims

1. A bead comprising a polymer comprising uronic acid units having mercapto groups, the mercapto groups partly or entirely forming a disulfide bond.

2. The bead according to claim 1 wherein the uronic acid unit having the mercapto group comprises a uronic acid residue and a residue of a compound represented by the formula (A1):

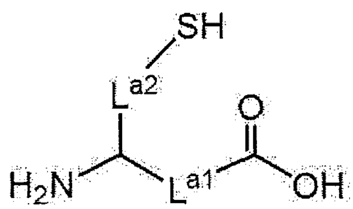

(A1)

wherein $L^{a1}$ is a single bond or a $C_{1-3}$ alkylene group and $L^{a2}$ is a $C_{1-4}$ alkylene group, or a compound represented by the formula (A2) :

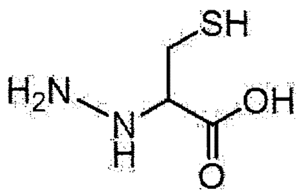

(A2)

bonded to each other via an amide bond.

3. The bead according to claim 1 or 2 wherein the uronic acid is at least one selected from the group consisting of galacturonic acid, mannuronic acid and guluronic acid.

4. The bead according to claim 1 or 2 wherein the uronic acid is at least one selected from the group consisting of mannuronic acid and guluronic acid, and the polymer comprising the uronic acid units having the mercapto groups is alginic acid having mercapto groups.

5. The bead according to claim 1 wherein the polymer comprising the uronic acid units having the mercapto groups is a polymer comprising galacturonic acid units having mercapto groups.

6. The bead according to claim 5 wherein the galacturonic acid unit having the mercapto group comprises a galacturonic acid residue and an amino acid residue having a mercapto group bonded to each other via an amide bond.

7. The bead according to claim 6 wherein the amino acid having the mercapto group is cysteine.

8. The bead according to any one of claims 5 to 7 wherein the polymer comprising the galacturonic acid units is polygalacturonic acid.

**9.** The bead according to any one of claims 1 to 8 wherein the polymer comprising the uronic acid units having the mercapto groups is further bonded to a compound represented by the formula (B):

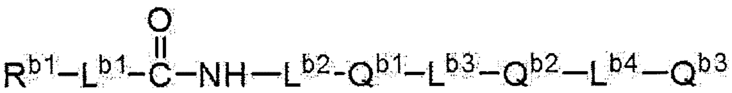 (B)

wherein $R^{b1}$ is a functional group capable of bonding to a mercapto group,
$L^{b1}$ and $L^{b2}$ are each independently a single bond or a $C_{1-6}$ alkylene group,
$Q^{b1}$ is a single bond, a phenylene group, or a $C_{4-8}$ cycloalkanediyl group,
$L^{b3}$ is a single bond or *-$(OCH_2CH_2)_n$-** (wherein * shows a bonding position to $Q^{b1}$, ** shows a bonding position to $Q^{b2}$, and n is an integer of 1 - 10),
$Q^{b2}$ is a divalent triazole ring group,
$L^{b4}$ is a single bond, a $C_{1-6}$ alkylene group, or a $C_{1-6}$ alkylene-oxy group, and
$Q^{b3}$ is an optionally substituted phenyl group, an optionally substituted monovalent 5- or 6-membered heterocyclic group, or an optionally substituted $C_{4-8}$ cycloalkyl group.

**10.** The bead according to claim 9 wherein the compound represented by the formula (B) is at least one selected from the group consisting of a compound represented by the formula (B1) :

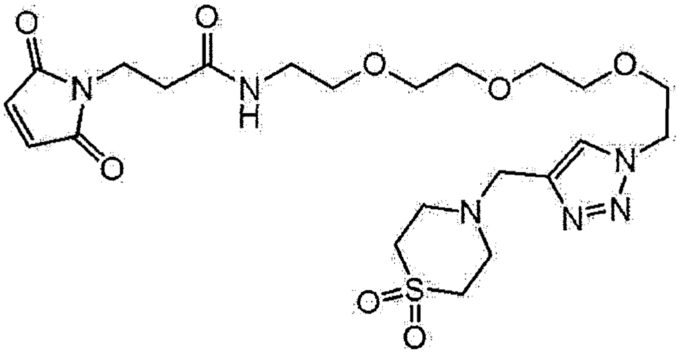 (B1)

,

a compound represented by the formula (B2):

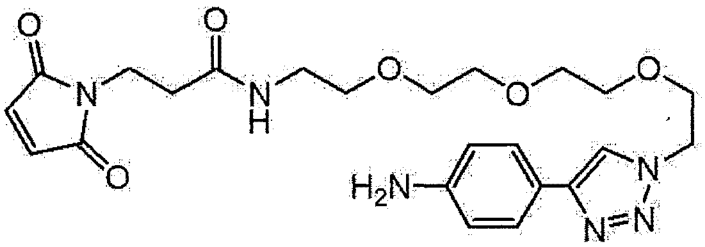 (B2)

,

and
a compound represented by the formula (B3):

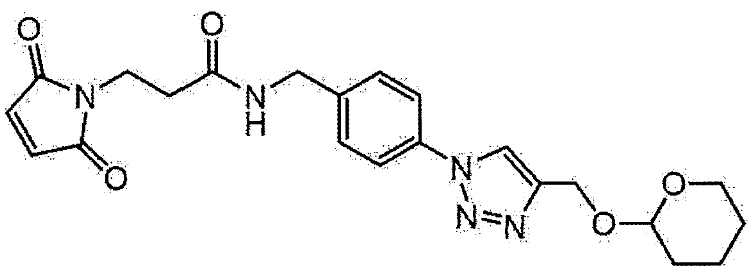 (B3)

.

**11.** The bead according to claim 9 wherein the compound represented by the formula (B) is a compound represented

by the formula (B1):

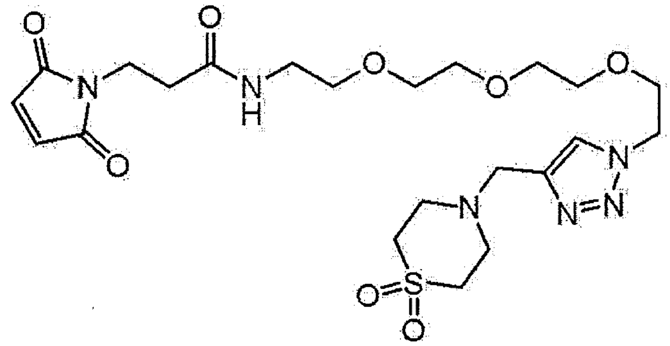

(B1)

.

**12.** The bead according to any one of claims 9 to 11 wherein the compound represented by the formula (B) is a compound having an inhibitory action on a foreign-body reaction.

**13.** The bead according to any one of claims 1 to 12 wherein a proportion of the uronic acid unit having the mercapto group in the total constitutional units of the polymer comprising the uronic acid units having the mercapto groups is 0.1 - 50 mol%.

**14.** The bead according to any one of claims 1 to 13 wherein a proportion of the mercapto group forming the disulfide bond in the total mercapto groups is 10 - 100 mol%.

**15.** The bead according to any one of claims 1 to 14 wherein a number average molecular weight of the polymer comprising the uronic acid units having the mercapto groups is 25,000 - 500,000.

**16.** The bead according to any one of claims 1 to 15 wherein the polymer comprises a divalent metal ion.

**17.** The bead according to claim 16 wherein the divalent metal ion is at least one selected from the group consisting of calcium ion, barium ion and strontium ion.

**18.** The bead according to any one of claims 1 to 17 further comprising a first layer and a second layer as outer layers, wherein the first layer is formed on the bead and the second layer is formed on the first layer.

**19.** The bead according to claim 18 wherein the uronic acid is at least one selected from the group consisting of mannuronic acid and guluronic acid,

the polymer comprising the uronic acid units having the mercapto groups is alginic acid having mercapto groups, and
the uronic acid unit having the mercapto group comprises a uronic acid residue and a residue of a compound represented by the formula (A1):

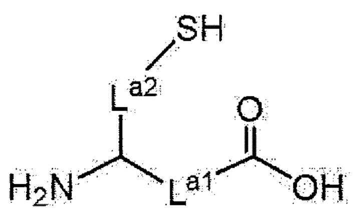

(A1)

wherein $L^{a1}$ is a single bond or a $C_{1-3}$ alkylene group and $L^{a2}$ is a $C_{1-4}$ alkylene group, or a compound represented by the formula (A2) :

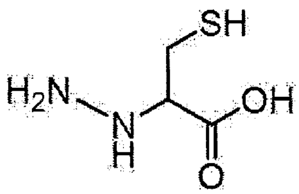 (A2)

bonded to each other via an amide bond.

**20.** The bead according to claim 19 wherein the compound represented by the formula (A1) or the compound represented by the formula (A2) is cysteine.

**21.** The bead according to any one of claims 18 to 20 wherein the first layer is formed from at least one selected from the group consisting of water-soluble chitosan and polyornithine.

**22.** The bead according to any one of claims 18 to 21 wherein the second layer is formed from at least one selected from the group consisting of polygalacturonic acid and polygalacturonic acid having mercapto groups.

**23.** The bead according to claim 22 wherein said at least one selected from the group consisting of the polygalacturonic acid and polygalacturonic acid having mercapto groups is further bonded to a compound represented by the formula (b):

$$H_2N\text{-}L^{b2}\text{-}Q^{b1}\text{-}L^{b3}\text{-}Q^{b2}\text{-}L^{b4}\text{-}Q^{b3} \qquad (b)$$

wherein $L^{b2}$ is a single bond or a $C_{1-6}$ alkylene group,
$Q^{b1}$ is a single bond, a phenylene group, or a $C_{4-8}$ cycloalkanediyl group,
$L^{b3}$ is a single bond or *- $(OCH_2CH_2)_n$-** (wherein * shows a bonding position to $Q^{b1}$, ** shows a bonding position to $Q^{b2}$, and n is an integer of 1 - 10),
$Q^{b2}$ is a divalent triazole ring group,
$L^{b4}$ is a single bond, a $C_{1-6}$ alkylene group, or a $C_{1-6}$ alkylene-oxy group, and
$Q^{b3}$ is an optionally substituted phenyl group, an optionally substituted monovalent 5- or 6-membered heterocyclic group, or an optionally substituted $C_{4-8}$ cycloalkyl group.

**24.** The bead according to claim 23 wherein the compound represented by the formula (b) is at least one selected from the group consisting of a compound represented by the formula (b1) :

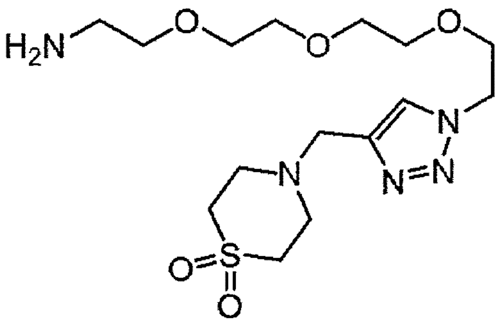 (b1)

,

a compound represented by the formula (b2):

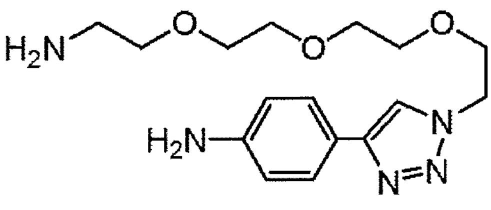 (b2)

,

and
a compound represented by the formula (b3):

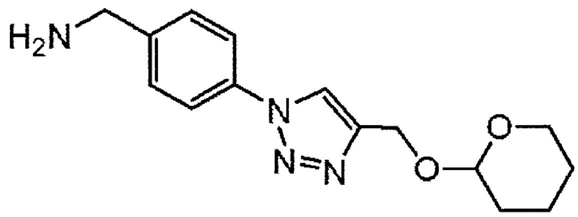

(b3)

.

**25.** The bead according to claim 23 wherein the compound represented by the formula (b) is a compound represented by the formula (b1):

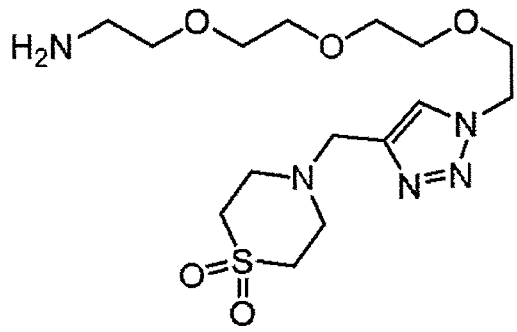

(b1)

.

**26.** The bead according to any one of claims 23 to 25 wherein the compound represented by the formula (b) is a compound having an inhibitory action on a foreign-body reaction.

**27.** The bead according to any one of claims 1 to 26 for use for enclosing a cell or a microorganism in the inside.

**28.** The bead according to any one of claims 1 to 26 enclosing the cell or microorganism in the inside.

**INTERNATIONAL SEARCH REPORT**

International application No. PCT/JP2018/005156

A. CLASSIFICATION OF SUBJECT MATTER
Int.Cl. C12N11/10(2006.01)i, C12N1/00(2006.01)i

According to International Patent Classification (IPC) or to both national classification and IPC

B. FIELDS SEARCHED

Minimum documentation searched (classification system followed by classification symbols)
Int.Cl. C12N11/10, C12N1/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
JSTPlus/JMEDPlus/JST7580(JDreamIII), CAplus/MEDLINE/EMBASE/BIOSIS/WPIDS(STN)

C. DOCUMENTS CONSIDERED TO BE RELEVANT

| Category* | Citation of document, with indication, where appropriate, of the relevant passages | Relevant to claim No. |
|---|---|---|
| X<br>Y | ISLAM, M. A. et al., "Mucoadhesive Alginate/poly(L-Lysine)/Thiolated Alginate Microcapsules for Oral Delivery of Lactobacillus Salivarius 29", J. Nanoscience Nanothechnology, 2011, vol. 11, no. 8, pp. 7091-7095, abstract, experimental details, fig. 1 | 1-4, 13-16, 27, 28<br>9-16, 18-28 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 May 2018 (08.05.2018) | 22 May 2018 (22.05.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/005156

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | TAHA, M. O. et al., "Synthesis of zinc-crosslinked thiolated alginic acid beads and their in vitro evaluation as potential enteric delivery system with folic acid as model drug", Pharmazie, 2005, vol. 60, pp. 736-742, abstract, Scheme 1 | 1-4, 13-16, 27, 28<br>9-16, 18-28 |
| X<br>Y | DESAI, R. M. et al., "Versatile click alginate hydrogels crosslinked via tetrazine-norbornene chemistry", 2015, vol. 50, pp. 30-37, abstract, materials and methods, fig. 3 | 1, 3, 4, 13-15, 27, 28<br>9-15, 18, 21-28 |
| X<br>Y | MAHOU, R. et al., "Tuning the Properties of Hydrogel Microspheres by Adding Chemical Cross-linking Functionality to Sodium Alginate", Chem. Mater., 2015, vol. 27, pp. 4380-4389, abstract, experimental section | 1-4, 13-17, 27, 28<br>9-28 |
| X<br>Y | SHARMA, R. et al., "Thiolated pectin: Synthesis, characterization and evaluation as a mucoadhesive polymer", Carbohydrate polymers, 2011, vol. 85, pp. 658-663, abstract, experimental | 1, 3, 5, 8, 13-17, 27, 28<br>9-18, 21-28 |
| X<br>Y | MAJZOOB, S. et al., "Pectin-cysteine conjugate: Synthesis and in-vitro evaluation of its potential for drug delivery", Journal of Pharmacy and Pharmacology, 2006, vol. 58, pp. 1601-1610, abstract, fig. 1 | 1-3, 5-8, 13-16, 27, 28<br>9-16, 18, 21-28 |
| Y | VEGAS, A. J. et al., "Combinatorial hydrogel library enables identification of materials that mitigate the foreign body response in primates", 2016, vol. 34, no. 3, pp. 345-352, ON LINE METHODS, Supplymentary Data, abstract, fig. 2, Supplementary Data | 9-28 |
| Y | JP 2006-089641 A (FUJI XEROX CO., LTD.) 06 April 2006, paragraph [0023] (Family: none) | 9-28 |
| Y | JP 2014-506926 A (WAKE FOREST UNIVERSITY HEALTH SCIENCES) 20 March 2014, examples & US 2014/0127308 A1, examples & WO 2012/121874 A1 & EP 2680862 A1 | 18-28 |
| Y | JP 1-127037 A (QUEEN'S UNIVERSITY AT KINGSTON) 19 May 1989, examples & US 4942129 A1, examples & EP 0301777 A1 | 18-28 |

**INTERNATIONAL SEARCH REPORT**

International application No. PCT/JP2018/005156

C (Continuation). DOCUMENTS CONSIDERED TO BE RELEVANT

| Category* | Citation of document, with indication, where appropriate, of the relevant passages | Relevant to claim No. |
|---|---|---|
| Y | HILLBERG, A. L. et al., "Improving alginate-poly-L-ornithine-alginate capsule biocompatibility through genipin crosslinking", Journal of Biomedical Materials research B: Applied Biomaterials, 2013, vol. 1018, no. 2, pp. 258-268, abstract, introduction | 18-28 |
| Y | JP 2012-525338 A (MICROPHARMA LIMITED) 22 October 2012, claims, paragraph [0063] & US 2011/0217368 A1, claims, paragraph [0074] & WO 2010/124387 A1 & EP 2419114 A1 | 18-28 |
| Y | MATTHEW, H. W. et al., "Complex Coacervate Microcapsules for Mammalian Cell Culture and Artificial Organ Development", Biotechnol. Prog., 1993, vol. 9, pp. 510-519, table 1 | 18-28 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2017027246 A
- JP 2017225903 A


### Non-patent literature cited in the description


- *JOURNAL OF BIOMEDICAL MATERIALS RESEARCH B: APPLIED BIOMATERIALS I,* February 2013, vol. 101B (2), 258-268
- *J Mater Sci: Mater Med,* 2013, vol. 24, 1375-1382
- *Nature Biotechnology,* 2016, vol. 34 (3), 345-352